# EUROPEAN PATENT APPLICATION

(11) **EP 0 864 567 A1**
(43) Date of publication of application: **16.09.1998**
(21) Application number: 96921117.6
(22) Date of filing: 28.06.1996
(51) Int. Cl.: C07D 251/18, C07D 405/12, A01N 43/68

(54) **TRIAZINE DERIVATIVES**

(30) Priority: 27.11.1995 JP 307394/95
(71) Applicant: IDEMITSU KOSAN COMPANY LIMITED, Tokyo 100-0005 (JP)
(72) Inventor: SAITO, Masatoshi, Sodegaura-shi, Chiba-ken 299-02 (JP); KUBOTA, Mineyuki, Sodegaura-shi, Chiba-ken 299-02 (JP); KOIKE, Kazuyoshi, Sodegaura-shi, Chiba-ken 299-02 (JP)
(74) Representative: Türk, Gille, Hrabal, Leifert
(86) International application number: JP9601799
(87) International publication number: WO9719936

(57) **Abstract**

The present invention relates to a triazine derivative of the general formula (I), 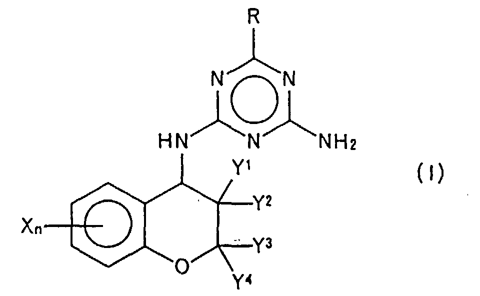 wherein each symbols are as defined in claims, a process for the production thereof, and a herbicide containing the triazine derivative of the above general formula (I) or a salt thereof as an active ingredient.

The above triazine derivative of the present invention is free from causing phytotoxicity on cotton and capable of selectively controlling a broad range of upland weeds including velvetleaf belonging to malvaceous weeds to which cotton also belongs at a low dosage, and the herbicide of the present invention, which contains the above triazine derivative as an active ingredient, is therefore remarkably useful as a herbicide for application in cotton fields.

## Description

### Technical Field

The present invention relates to a novel triazine derivative, a process for the production thereof and a herbicide containing the above triazine derivative as an active ingredient. More specifically, it relates to a triazine derivative which causes no phytotoxicity on cotton and can selectively control, at a low dosage, a broad range of upland weeds including velvetleaf belonging to malvaceous weeds to which cotton also belongs, a process for effectively producing the above triazine derivative and a herbicide containing the above triazine derivative as an active ingredient.

### Technical Background

Herbicides are very important chemicals for labor-saving in weed control and improving the productivity of agricultural and horticultural crops. Herbicides have been therefore actively studied and developed for many years, and a diversity of herbicides have been and are practically used. Even today, however, it is still desired to develop novel herbicides having herbicidal properties, particularly chemicals which can selectively control object weeds at a low dosage without causing phytotoxicity on cultivated crops.

On the other hand, it is known that annual gramineous weeds such as large crabgrass and annual broad-leaved weeds such as morning glory, slender amaranth, cocklebur and velvetleaf occur in cotton fields. In cotton planting, it is very important to control these weeds effectively at a low dosage in view of environmental pollution and without causing phytotoxicity on cotton. Since cotton comes under malvaceous weeds, particualrly, a chemical having herbicidal activity on velvetleaf which also comes under malvaceous weeds is liable to cause phytotoxicity on cotton. It is therefore an essential object to develop a chemical which has high herbicidal activity on velvetleaf and has excellent inter-genus selectivity between cotton and velvetleaf.

Various compounds have been and are known as triazine-containing herbicides. For example, it is known that 2-chloro-4,6-bis(alkylamino)-s-trizaine derivatives have broad herbicidal spectra and are useful as herbicides. However, these known triazine-containing herbicides requires large dosages for attaining high herbicidal efficacy. And, these chemicals are causing environmental problems that they contaminate groundwater, etc., due to their high percolation through soil.

### Disclosure of the Invention

Under the circumstances, the present invention aims at providing a herbicidal compound which exhibits a sufficient herbicidal efficacy at a low dosage and is environmentally safe and which has excellent inter-genus selectivity between cotton and velvetleaf.

For achieving the above object, the present inventors have made diligent studies and have found that a novel triazine derivative in which a phenyl-group-fused carbon-chain cyclic group and a trizine ring are bonded to each other, or a chroman ring and a triazine ring are bonded to each other, through an amino group causes no phytotoxicity on cotton and exhibits excellent herbicidal activity on velvetleaf which is a malvaceous weed as well as cotton.

That is, the gist of the present invention is a triazine derivative of the general formula (I), 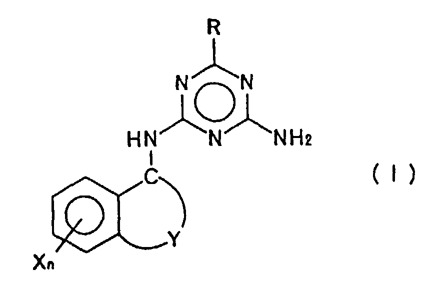
wherein X is a halogen atom, a hydroxyl group, a cyano group, a C₁-C₆ alkyl group, a C₁-C₄ alkoxy group, a C₁-C₄ alkylthio group, a C₁-C₄ alkylsulfonyl group, a C₁-C₆ haloalkyl group, a C₁-C₄ haloalkoxy group, a phenyl-substituted C₁-C₄ alkyl group, a phenyl group or a phenoxy group, provided that when the number of X is plural, plural substituents X may be the same as, or different from, each other or two vicinal substituents X may form a saturated or unsaturated five-membered or six-membered ring together with a carbon-carbon bond in a benzene ring, n is an integer of 0 or 1 to 4,
R is
   (1) a C₁-C₆ alkyl group or
   (2) a substituted C₁-c₆ alkyl group having 1 to 13 substituents of one or two kinds selected from the class consisting of
      i) a halogen atom
      ii) a hydroxyl group and
      iii) a C₁-C₈ alkoxy group whose alkyl portion may contain a hetero atom, and
Y is a C₂-C₄ alkylene group which may be substituted with 1 to 8 C₁-C₆ alkyl groups or a divalent group of the formula (a), 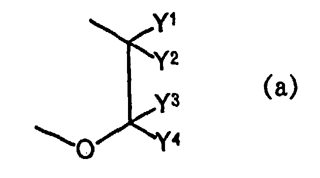
in which each of Y¹ to Y⁴ is independently a hydrogen atom or a C₁-C₄ alkyl group.

Further, the gist of the present invention is a process for the production of a triazine derivative of the general formula (I), 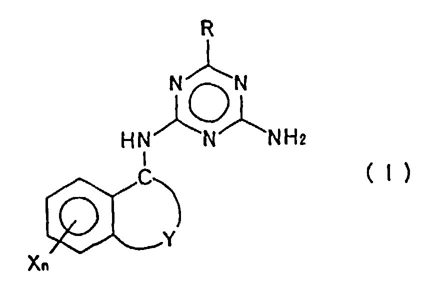
wherein X, n, Y and R are as defined above,
which comprises reacting a compound of the general formula (II), 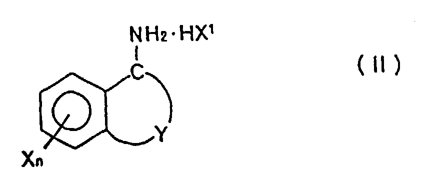
wherein X, n and Y are as defined above and X¹ is a halogen atom,
with cyanoguanidine of the formula (III), 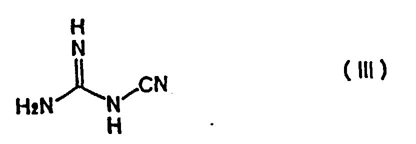 and then reacting the reaction product with an ester of of the general formula (IV),

RCOOR¹ (IV)

wherein R is as defined above and R¹ is a C₁-C₄ alkyl group.

Further, the gist of the present invention is a herbicide containing the triazine derivative of the above general formula (I) or a salt thereof as an active ingredient.

### Best Modes for Practicing the Invention

The triazine derivative of the present invention (to be sometimes referred to as "triazine derivative (I) hereinafter) is a compound having the following general formula (I).

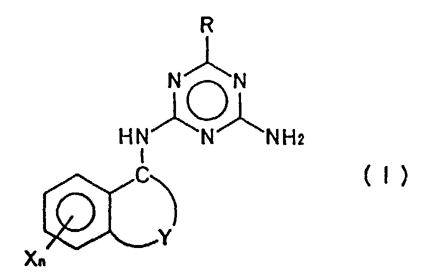

In the above general formula (I), X is a halogen atom, a hydroxyl group, a cyano group, a C₁-C₆ alkyl group, a C₁-C₄ alkoxy group, a C₁-C₄ alkylthio group, a C₁-C₄ alkylsulfonyl group, a C₁-C₆ haloalkyl group, a C₁-C₄ haloalkoxy group, a phenyl-group-substituted C₁-C₄ alkylgroup, a phenyl group or a phenoxy group.

When the above X is a halogen atom, specific examples of the halogen atom include a chlorine atom, a bromine atom, a fluorine atom and an iodine atom. The halogen atom is preferably a chlorine atom, a fluorine atom or a bromine atom.

When X is a C₁-C₆ alkyl group, specific examples thereof include methyl, ethyl, propyl, butyl, pentyl and hexyl. Those alkyl groups having 3 to 6 carbon atoms may be linear or branched. Further, the C₁-C₆ alkyl group may be a cycloalkyl group per se, such as cyclopropyl, cyclobutyl, cyclpentyl and cyclohexyl. Further, it may be an alkyl group containing a cycloalkyl group, such as cyclopropylmethyl. The above C₁-C₆ alkyl group Is preferably methyl, ethyl, i-propyl or t-butyl, particularly preferably methyl.

When X is a C₁-C₄ alkoxy group, specific examples thereof include methoxy, ethoxy, propoxy and butoxy. The alkoxy group having 3 or 4 carbon atoms may be linear or branched. Specific examples of the C₁-C₄ alkoxy group also include cyclopropoxy on which methyl may be substituted and cyclobutoxy. The C₁-C₄ alkoxy group is preferably methoxy.

When X is a C₁-C₄ alkylthio group, specific examples thereof include -SCH₃, -SC₂H₅, -SC₃H₇ and -SC₄H₉ groups. Of these, the alkylthio group having 3 or 4 carbon atoms may be linear or branched. The C₁-C₄ alkylthio group is preferably -SCH₃.

When X is a C₁-C₄ alkylsulfonyl group, specific examples thereof include -SO₂CH₃, -SO₂C₂H₅, -SO₂C₃H₇ and -SO₂C₄H₉ groups. Of these, the alkylsulfonyl group having 3 or 4 carbon atoms may be linear or branched. The C₁-C₄ alkylsulfonyl group is preferably -SO₂CH₃.

The C₁-C₆ haloalkyl group as one embodiment of X is a group formed by replacing 1 to 13 hydrogen atoms bonding to carbon atom(s) of the above C₁-C₆ alkyl group with the above halogen atom(s). Specific examples of the C₁-C₆ haloalkyl group include -CF₃, -CH₂F, -CCl₃ and -CH₂CF₃ groups. The C₁-c₆ haloalkyl group is preferably -CF₃.

The C₁-C₄ haloalkoxy group as one embodiment of X is a group is a group formed by replacing 1 to 9 hydrogen atoms bonding to carbon atom(s) of the above C₁-C₄ alkoxy group with the above halogen atom(s). Specific examples of the C₁-C₄ haloalkoxy group include -OCF₃, -OCCl₃ and -OCH₂F groups. The C₁-C₄ haloalkoxy group is preferably -CF₃.

The phenyl-group-substituted C₁-C₄ alkyl group as one embodiment of X is a group formed by replacing one or at least two hydrogen atoms bonding to carbon atom(s) of a C₁-C₄ alkyl group with phenyl group(s). The above C₁-C₄ alkyl group includes methyl, ethyl, propyl and butyl, and of these, the propyl and the butyl may be linear or branched. Specific examples of the phenyl-group-substituted C₁-C₄ alkyl group include groups of -CH₂Ph (Ph represents a phenyl group) and -CH₂CH₂Ph, and it is preferably -CH₂Ph.

The position on which X is substituted is as follows. When Y to be explained later is a C₂-C₄ alkylene group which may be substituted with 1 to 8 C₁-C₄ alkyl groups, X may be positioned on any carbon of an aromatic group fused with a carbon-chain ring containing Y. Preferably, of positions ① to ④ shown in the following general formula (I), on which X can be substituted, the position ②, the position ③ or the position ④, both the positions ② and ④ or both the positions ③ and ④ is/are preferred.

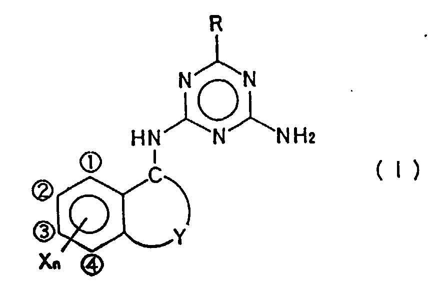

When Y to be explained later is a divalent group of the formula (a), 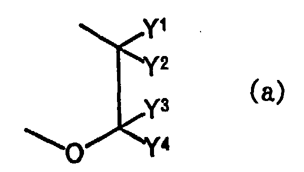
wherein Y¹ to Y⁴ are as defined above,
X can be substituted on any one of four carbon atoms at 5th to 8th positions of a chroman ring.
In the above general formula (I), n which represents the number of substituent(s) X is an integer of 0 or 1 to 4, preferably 0, 1 or 2. When n is 2 to 4, i.e., when the number of substituent(s) X is 2 to 4, 2 or more substituents X may be the same as, or different from, each other.

Further, two vicinal substituents X may form a saturated or unsaturated five-membered or six-membered ring together with a carbon-carbon bond of a benzene ring. That is, the substituents X may form an indene ring, an indane ring, a naphthalene ring or a tetralin ring together with a benzene ring to which the substituents X bond.

In the above general formula (I), Y is a C₂-C₄ alkylene group which may be substituted with 1 to 8 C₁-C₄ alkyl group(s) or a divalent group of the formula (a).

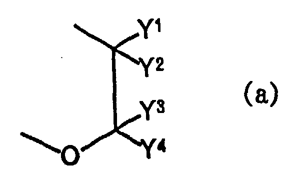

When Y is the above C₂-C₄ alkylene group, particularly, a group in which a carbon-chain ring is fused with a phenyl group is a so-called indanyl group when Y is an ethylene group (C₂ alkylene group), and it is a so-called tetralinyl group when Y is a propylene group (C₃ alkylene group).

Specific examples of the C₁-C₄ alkyl group(s) which may be substituted on the C₂-C₄ alkylene group as Y are the same as C₁-C₄ alkyl groups of the C₁-C₆ alkyl group in X. The above C₁-C₄ alkyl group is preferably methyl. The number of the C₁-C₄ alkyl group(s) which may be substituted on the C₂-C₄ alkylene group as Y is 1 to 8. The C₁-C₄ alkyl group(s) which may be substituted on the alkylene group as Y may be substituted on any hydrogen atom(s) of four hydrogen atoms of an ethylene group when the alkylene group is an ethylene group (C₂), on any hydrogen atoms of six hydrogen atoms of a propylene group when the alkylene group is a propylene group (C₃) or on any hydrogen atoms of eight hydrogen atoms of a butylene group when the alkylene group is a butylene group (C₄).

When Y is a divalent group of the above formula (a), the triazine derivative of the present invention can be represented by the following general formula (I'), 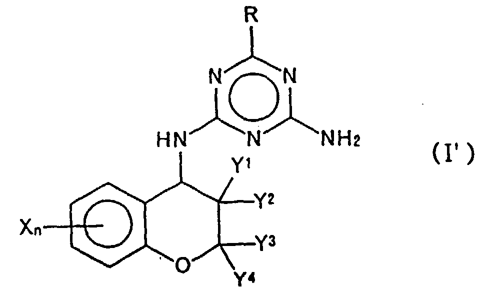
wherein X, n and R are as defined in the general formula (I).

In the above general formula (I'), each of Y¹ to Y⁴ is independently a hydrogen atom or a C₁-C₄ alkyl group, preferably a hydrogen atom or methyl.

In the general formula (I), R is
(1) a C₁-C₆ alkyl group or
(2) a substituted C₁-C₆ alkyl group having 1 to 13 substituents of one or two kinds selected from the class consisting of
   i) a halogen atom
   ii) a hydroxyl group and
   iii) a C₁-C₈ alkoxy group whose alkyl portion may contain a hetero atom.

Specific examples of the C₁-C₆ alkyl group in (1) include those explained concerning X, and the C₁-C₆ alkyl group is preferably t-butyl.

Specific examples of i) the halogen atom as a substituent of one kind in the substituted C₁-C₆ alkyl group in (2) include those explained concerning X, and the halogen atom is preferably a fluorine atom or a chlorine atom. Therefore, specific examples of a halogen-atom-substituted C₁-C₆ alkyl group included in the substituted C₁-C₆ alkyl group in (2) are -CF₃, -CCl₃, -CH₂F, -CH₂Cl, -CH₂Br, -C₂F₅, -CH₂CH₂F, -CHF(CH₃), -CHCl(CH₃), -CHBr(CH₃), -CHF(CF₃), -CF(CH₃)₂, -CCl(CH₃)₂, -CBr(CH₃)₂, -CHF(CH₂CH₃), -CHCl(CH₂CH₃) and -CHBr(CH₂CH₃) groups. The halogen-atom-sbustituted C₁-C₆ alkyl group is preferably -CF₃, -CHF(CH₃), -CHF(CF₃), -CF(CH₃)₂ or -CCl(CH₃)₂.

Specific examples of a hydroxyl-substituted C₁-C₆ alkyl group included in the substituted C₁-C₆ alkyl group in (2) are -CH₂OH, -C₂H₄OH, -CH(OH)CH₃, -CH(OH)C₂H₅, -C(CH₃)₂OH and -C(CH₃)₂CH₂OH groups, and the hydroxyl-substituted C₁-C₆ alkyl group is preferably -CH(OH)C₂H₅.

Specific examples of iii) the C₁-C₈ alkoxy group whose alkyl portion may contain a hetero atom, as a substituent of another kind in the substituted C₁-C₆ alkyl group in (2), include aliphatic alkoxy groups such as methoxy, ethoxy, propoxy, butoxy, pentoxy, hexanoxy, heptanoxy and octanoxy;
alicyclic alkoxy groups such as 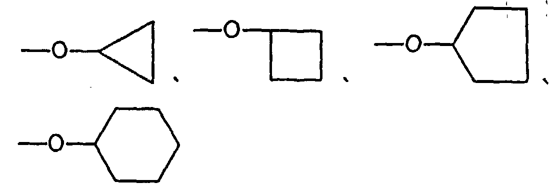 alicyclic-aliphatic alkoxy groups such as 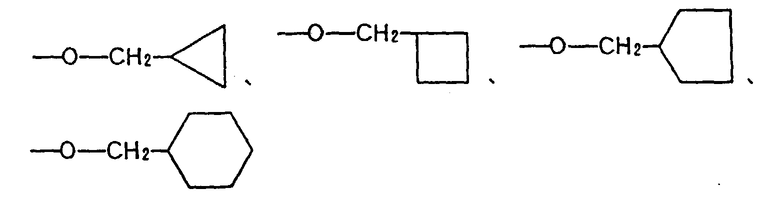 and groups in which a heterocyclic group (heterocyclic group refers to a cyclic group containing at least one hetero atoms (e.g., oxygen atom, nitrogen atom, sulfur atom, or the like)) and an oxygen atom bond to each other, such as

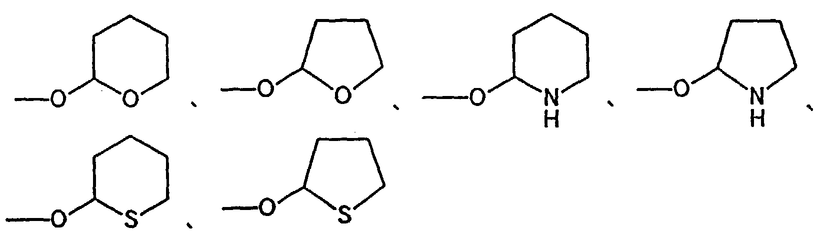

The groups in which a heterocyclic group and an oxygen atom bond to each other is a group in which an oxygen atom bonds to the above heterocyclic group so as to form an ether bond.

Specific examples of the C₁-C₈ alkoxy-substituted C₁-C₆ alkyl group which is included in the substituted C₁-C₆ alkyl group in (2) and contains, as a substituent, the C₁-C₈ alkoxy group whose alkyl portion may contain a hetero atom, preferably include aliphatic-alkoxy-substituted alkyl groups such as -CH₂-OCH₃, -CH₂OC(CH₃)₃, -C₂H₄-OCH₃, -C₃H₆-OCH₃, -C₃H₆-OC₂H₅, -CH(CH₃)OCH₃, -CH(CH₂CH₃)OCH₃, -CH(CH₃)CH₂OCH₃ and -C(CH₃)₂CH₂OCH₃ groups; and alkyl groups on which a combination of a heterocyclic group and an oxygen atom is substituted, such as

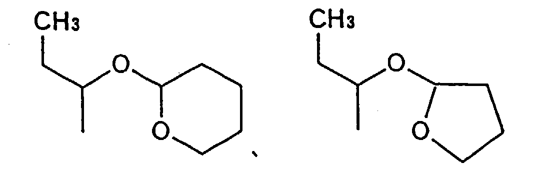

The above substituted alkyl group having a hetero ring is preferably

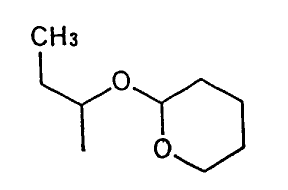

The substituted C₁-C₆ alkyl group refers to a C₁-C₆ alkyl group having substituent(s) of one or two kinds selected from the above three substituents i), ii) and iii) is/are substituted. The total number of the substituent(s) is 1 to 13. Specific examples of the C₁-C₆ alkyl group having substituents of two kinds include -CH(CF₃)OH, -CH(CF₃)OCH₃ and -CF₂OCH₃ groups.

The process for the production of the triazine derivative (I), provided by the present invention, comprises a reaction in a first step in which a compound of the general formula (II), 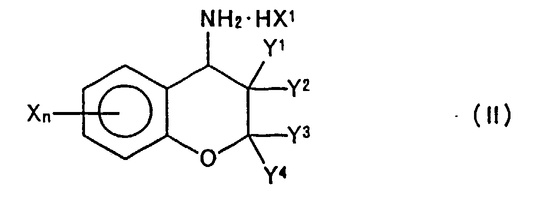
wherein X, n and Y are as explained in the above triazine derivative (I), and X¹ is a halogen atom,
and cyanoguanidine of the formula (III) 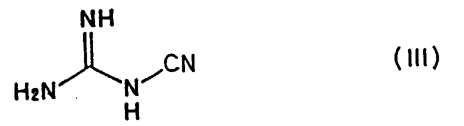 are allowed to react, to bond an amino group of the compound (II) and a cyano group of the cyanoguanidine (III) to each other; and
a reaction in a second step in which the reaction product is then reacted with an ester of the general formula (IV),

RCOOR¹ (IV)

wherein R is as explained in the above triazine derivative (I) and R¹ is a C₁-C₄ alkyl group,
in the presence of a catalyst.

The process for the production of the triazine derivative, provided by the present invention, will be shown by a reaction scheme below.

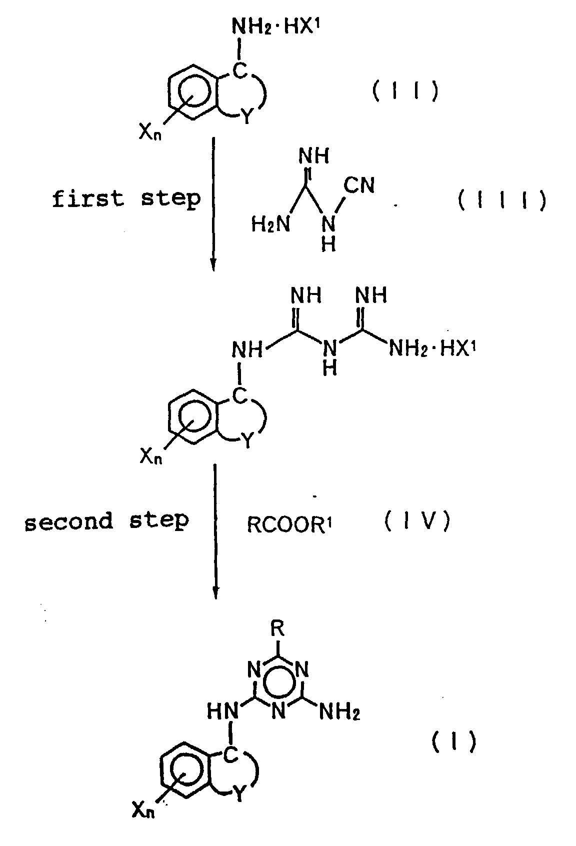

The reaction in the first step may be carried out in the absence or presence of a solvent. When the reaction is carried out in the presence of a solvent, the solvent can be selected from alcohols such as methanol, ethanol and isopropanol; ketones such as acetone, methyl ethyl ketone and cyclohexanone; aliphatic hydrocarbons such as n-hexane, n-heptane and n-decane; cyclic hydrocarbons such as benzene, decalin and alkylnaphthalene; chlorinated hydrocarbons such as carbon tetrachloride, methylene dichloride, chlorobenzene and dichlorobenzene; ethers such as tetrahydrofuran and dioxane; and further, kerosene. Aliphatic hydrocarbons are preferred, and n-decane is particularly preferred.

Preferably, a salt of the amine derivative (II) and the cyanoguanidine (III) are reacted in an equivalent ratio.

Specific examples of an acid (HX¹) for forming the salt of the amine derivative (II) include hydrochloric acid (HCl), hydrobromic acid (HBr) and hydrofluoric acid (HF), and hydrochloric acid (HCl) is preferred.

Although not specially limited, the reaction temperature is generally 80 to 200°C, preferably 120 to 150°C. The reaction time is generally 2 to 15 hours, preferably approximately 4 to 7 hours.

The reaction in the second step is preferably carried out in the presence of a catalyst. The catalyst that can be used in this reaction includes, for example, alkoxides such as sodium methoxide, sodium ethoxide and magnesium diethoxide; inorganic bases such as sodium phosphate, potassium carbonate, sodium hydroxide and potassium hydroxide; and organic bases such as 1,8-diazabicyclo[5,4,0]-7-undecene (DBU), 1,5-diazabicyclo[4,3,0]-5-nonene (DBN), triethylamine and pyridine. Sodium methoxide and sodium ethoxide are preferred. The amount of the base based on the amine derivative (II) is generally 1.1 to 10 equivalent amount, preferably 1.5 to 4 equivalent amount.

The amount of the ester (IV) used in the above reaction is generally 1 to 10 equivalent amount, preferably 1 to 4 equivalent amount, based on the amine derivative (II).

Preferably, the above reaction is carried out in the presence of a solvent. The solvent that can be used in the above reaction includes, for example, alcohols such as methanol, ethanol and isopropanol; ketones such as acetone, methyl ethyl ketone and cyclohexanone; aliphatic hydrocarbons such as n-hexane, n-heptane and n-decane; cyclic hydrocarbons such as benzene, decalin and alkylnaphthalene; chlorinated hydrocarbons such as carbon tetrachloride, methylene dichloride, chlorobenzene and dichlorobenzene; and ethers such as tetrahydrofuran and dioxane. Alcohols are preferred, and methanol and ethanol are particularly preferred.

In the above reaction, the reaction temperature is generally -10 to 100°C, preferably 0 to 70°C. The reaction time is generally 2 to 30 hours, preferably approximately 5 to 15 hours.

After completion of the reaction, according to a conventional method, a reaction mixture is poured into water, and extracted with an organic solvent such as ethyl acetate. An obtained organic layer is dehydrated with a dehydrating agent such as anhydrous sodium sulfate, and the organic solvent is removed by means of distilling it under reduced pressure or some other means. An obtained residue is purified by means of silica gel column chromatography or some other means, whereby the intended triazine derivative (I) can be isolated in the form of a crystal.

The hearbicide containing the triazine derivative (I) or its salt of the present invention as an active ingredient, provided by the present invention, will be explained below.

The herbicide of the present invention contains the novel triazine derivative of the general formula (I), provided by the present invention, or a salt thereof as an active ingredient. These compounds are used by mixing them with a liquid carrier such as a solvent or a solid carrier such as a mineral fine powder and preparing the resultant mixtures in the form of a wettable powder, an emulsifiable concentrate, a dust or granules. When the above preparations are formed, a surfactant can be added for imparting the above compounds with emulsifiability, dispersibility or spreadability.

When the herbicide of the present invention is used in the form of a wettable powder, generally, 10 to 55 % by weight of the triazine derivative (I) or the salt thereof, provided by the present invention, 40 to 88 % by weight of a solid carrier and 2 to 5 % by weight of a surfactant are mixed to prepare a composition, and the composition can be used.

When the herbicide of the present invention is used in the form of an emulsifiable concentrate, generally, it is sufficient to prepare a composition by mixing 20 to 50 % by weight of the triazine derivative (I) or the salt thereof, provided by the present invention, 35 to 75 % by weight of a solvent and 5 to 15 % by weight of a surfactant.

When the herbicide of the present invention is used in the form of a dust, generally, it is sufficient to prepare a composition by mixing 1 to 15 % by weight of the triazine derivative (I) or the salt thereof, provided by the present invention, 80 to 97 % by weight of a solid carrier and 2 to 5 % by weight of a surfactant.

Further, when the herbicide of the present invention is used in the form of granules, generally, it is sufficient to prepare a composition by mixing 1 to 15 % by weight of the triazine derivative (I) or the salt thereof, provided by the present invention, 80 to 97 % by weight of a sold carrier and 2 to 5 % by weight of a surfactant.

The above solid carrier is selected from fine mineral powders, and examples of the mineral fine powders include oxides such as diatomaceous earth and slaked lime, phosphates such as apatite, sulfates such as gypsum, and silicates such as talc, pyroferrite, clay, kaolin, bentonite, acid clay, white carbon, powdered quartz and powdered silica.

The solvent is selected from organic solvents. Specific examples of the solvent include aromatic hydrocarbons such as benzene, toluene and xylene, chlorinated hydrocarbons such as o-chlorotoluene, trichloroethane and trichloroethylene, alcohols such as cyclohexanol, amyl alcohol and ethylene glycol, ketones such as isophorone, cyclohexanone and cyclohexenyl-cyclohexanone, ethers such as butyl cellosolve, diethyl ether and methyl ethyl ether, esters such as isopropyl acetate, benzyl acetate and methyl phthalate, amides such as dimethylformamide, and mixtures of these.

Further, the surfactant can be selected from anionic surfactants, nonionic surfactants, cationic surfactants and amphoteric surfactants (amino acid and betaine).

The herbicide of the present invention may contain, as an active ingredient, other herbicidally active component as required in combination with the triazine derivative (I) or its salt. The "other" herbicidally active component includes known herbicides such as phenoxy-, diphenyl ether-, triazine-, urea-, carbamate-, thiolcarbamate-, acid anilide-, pyrazole-, phosphoric acid-, sulfonylurea- and oxadiazone-containing herbicides, and it can be properly selected from these herbicides as required.

Further, the herbicide of the present invention may be used as a mixture with any one of insecticides, bactericides, plant growth regulators and fertilizers.

The present invention will be specifically explained with reference to Examples and Herbicide Examples hereinafter, while the present invention shall not be limited thereto.

### (Example 1)

0.95 Gram (5.2 mmol) of 1-aminotetralin hydrochloride and 0.44 g (5.2 mmol) of cyanoguanidine were added to 20 ml of n-decane, and the mixture was stirred under heat at 135°C for 6 hours. After completion of the reaction, the reaction mixture was cooled, a formed precipitate was recovered by filtration and washed with 5 ml of n-hexane three times, and the solvent was removed under reduced pressure to give a solid. 1 Gram of the obtained solid was dissolved in 25 ml of absolute methanol, and to the resultant solution was added 1.9 g (10 mmol) of a 28 wt% sodium methoxide/methanol solution at room temperature. Further, 1.2 g (10 mmol) of ethyl α-fluoropropionate was dropwise added thereto, and the mixture was stirred at room temperature for 12 hours. After completion of the reaction, the reaction mixture was poured into 100 ml of water, and extraction was carried out with 50 ml of ethyl acetate three times. An obtained ethyl acetate layer was dried over anhydrous sodium sulfate, and the ethyl acetate was distilled off under reduced pressure. The resultant residue was purified by silica gel column chromatography (developer solution: hexane/ethyl acetate = 1/1 (volume ratio)) to give 0.68 g of 2-amino-4-(α-fluoroethyl)-6-(1'-tetralinylamino)-s-triazine as an end product in the form of a white crystal. Table 1 to be described later shows the structural formulae of the salt of a cycloalkylamine derivative and the ester both of which are used as raw materials and the structural formulae of the obtained triazine derivative and the yield thereof. Table 8 to be described later shows IR and NMR data of the obtained triazine derivative.

### (Examples 2 - 4)

Triazine derivatives as end products were obtained in the same manner as in Example 1 except that the ethyl α-fluoropropionate was replaced with esters shown in Table 1. Table 1 to be described later shows the structural formulae of the salt of a cycloalkylamine derivative and the esters both of which are used as raw materials and the structural formulae of the obtained triazine derivatives and the yields thereof. Table 8 to be described later shows IR and NMR data of the obtained triazine derivatives.

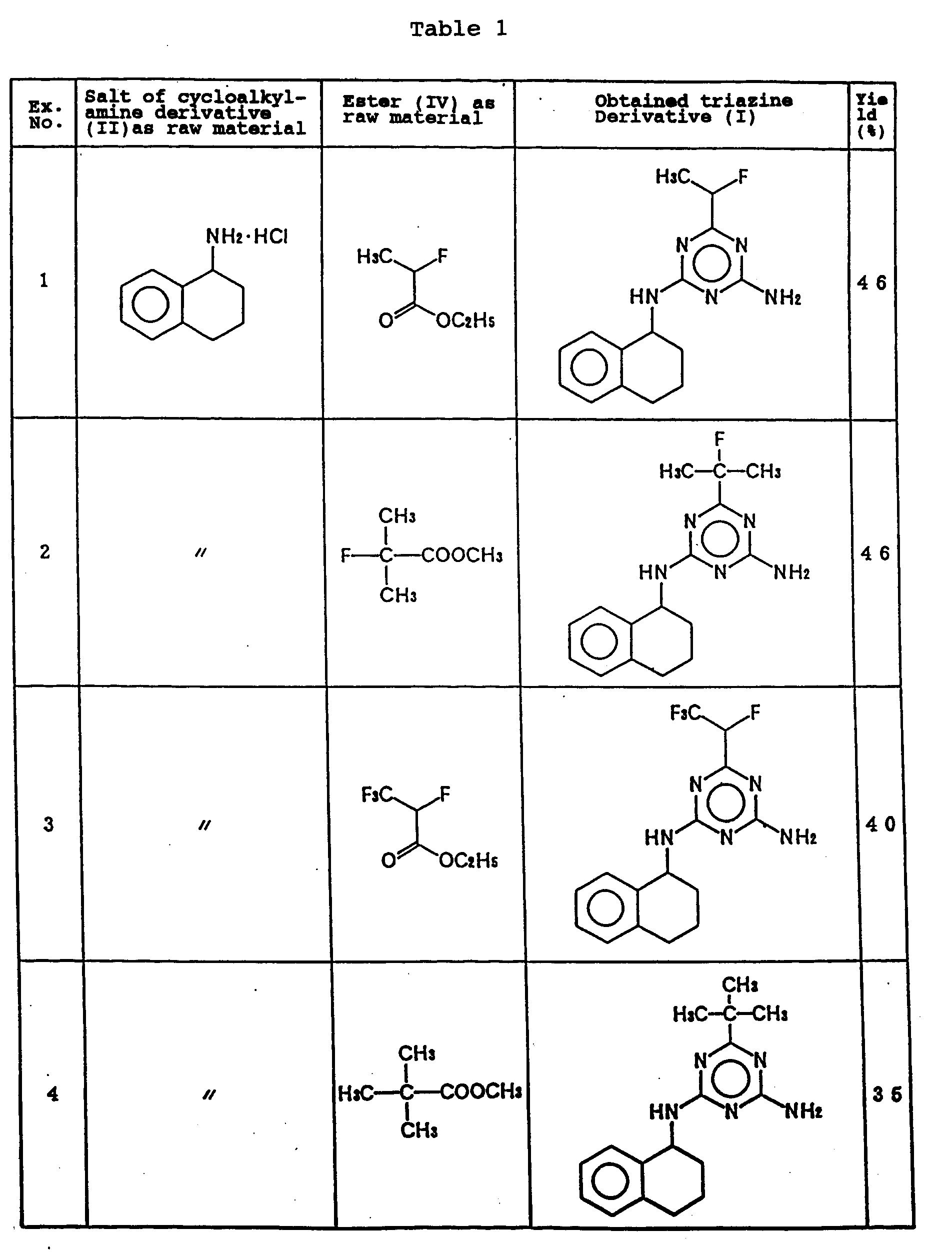

### (Examples 5 - 8)

Triazine derivatives as end products were obtained in the same manner as in Example 1 except that the 1-aminotetralin hydrochloride was replaced with salts of cycloalkylamine derivatives shown in Table 2. Tables 2 and 3 to be described later show the structural formulae of the salts of cycloalkylamine derivatives and the esters both of which are used as raw materials, the structural formulae of the obtained triazine derivatives and the yields thereof. Table 8 to be described later shows IR and NMR data of the obtained triazine derivatives.

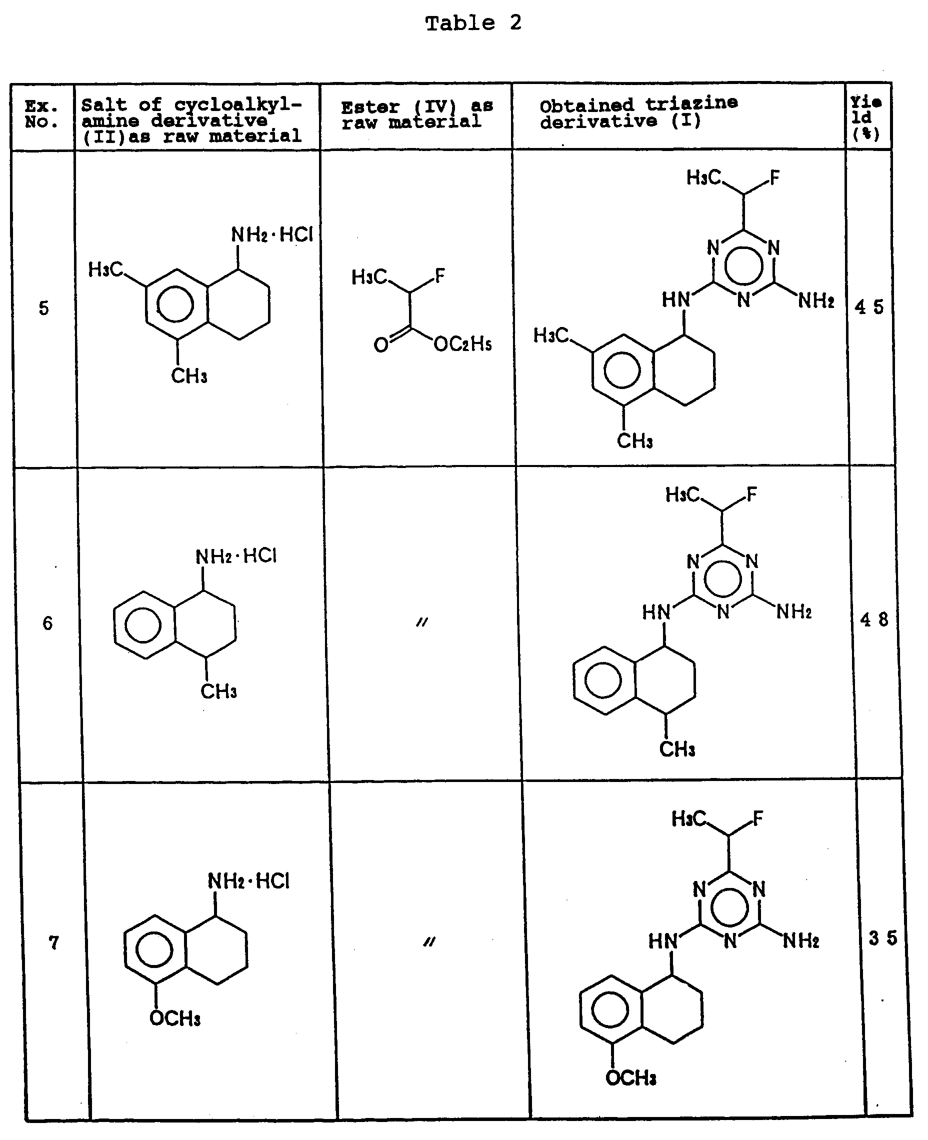

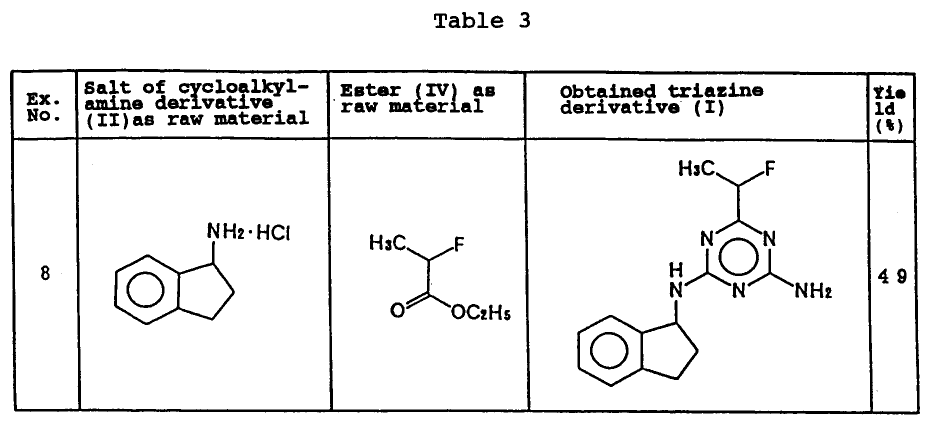

### (Example 9)

1.1 Grams (5.6 mmol) of 1-amino-2-methyltetralin hydrochloride and 0.48 g (5.6 mmol) of cyanoguanidine were added to 20 ml of n-decane, and the mixture was stirred under heat at 135°C for 6 hours. After completion of the reaction, the reaction mixture was cooled, a formed precipitate was recovered by filtration and washed with 5 ml of n-hexane three times, and the solvent was removed under reduced pressure to give a solid. 1 Gram of the obtained solid was dissolved in 25 ml of absolute methanol, and to the resultant solution was added 2.5 g (13 mmol) of a 28 wt% sodium methoxide/methanol solution at room temperature. Further, 1.85 g (13 mmol) of ethyl trifluoroacetate was dropwise added thereto, and the mixture was stirred at room temperature for 12 hours. After completion of the reaction, the reaction mixture was poured into 100 ml of water, and extraction was carried out with 50 ml of ethyl acetate three times. An obtained ethyl acetate layer was dried over anhydrous sodium sulfate, and the ethyl acetate was distilled off under reduced pressure. The resultant residue was purified by silica gel column chromatography (developer solution: hexane/ethyl acetate = 1/1 (volume ratio)) to give 1.0 g of 2-amino-4-trifluoromethyl-6-(2'-methyl-1'-tetralinylamino)-s-triazine as an end product in the form of a white crystal. Table 4 to be described later shows the structural formulae of the salt of a cycloalkylamine derivative and the ester both of which are used as raw materials, the structural formula of the obtained triazine derivative and the yield thereof. Table 8 to be described later shows IR and NMR data of the obtained triazine derivative.

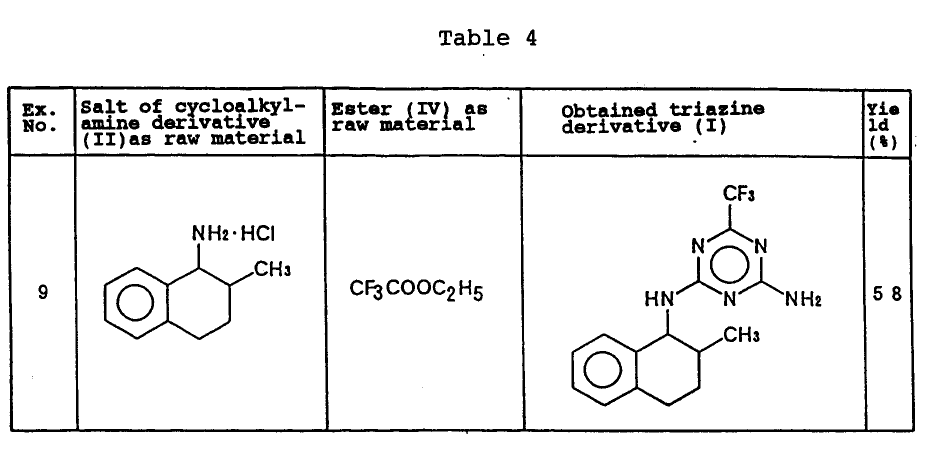

### (Example 10)

0.95 Gram (5.6 mmol) of 1-aminoindan hydrochloride and 0.48 g (5.6 mmol) of cyanoguanidine were added to 20 ml of n-decane, and the mixture was stirred under heat at 135°C for 6 hours. After completion of the reaction, the reaction mixture was cooled, a formed precipitate was recovered by filtration and washed with 5 ml of n-hexane three times, and the solvent was removed under reduced pressure to give a solid. 1 Gram of the obtained solid was dissolved in 25 ml of absolute methanol, and to the resultant solution was added 2.5 g (13 mmol) of a 28 wt% sodium methoxide/methanol solution at room temperature. Further, 1.56 g (13 mmol) of methyl α-fluoroisobutyrate was dropwise added thereto, and the mixture was stirred at room temperature for 12 hours. After completion of the reaction, the reaction mixture was poured into 100 ml of water, and extraction was carried out with 50 ml of ethyl acetate three times. An obtained ethyl acetate layer was dried over anhydrous sodium sulfate, and the ethyl acetate was distilled off under reduced pressure. The resultant residue was purified by silica gel column chromatography (developer solution: hexane/ethyl acetate = 1/1 (volume ratio)) to give 0.99 g of 2-amino-4-(α-fluoro-α-methylethyl)-6-(1'-indanylamino)-s-triazine as an end product in the form of a white crystal. Table 5 to be described later shows the structural formulae of the salt of a cycloalkylamine derivative and the ester both of which are used as raw materials, the structural formula of the obtained triazine derivative and the yield thereof. Table 8 to be described later shows IR and NMR data of the obtained triazine derivative.

### (Examples 11 - 14)

Triazine derivatives as end products were obtained in the same manner as in Example 10 except that the methyl α-fluoroisobutyrate was replaced with esters shown in Tables 5 and 6. Tables 5 and 6 be described later show the structural formulae of the salt of a cycloalkylamine derivative and the esters both of which are used as raw materials and the structural formulae of the obtained triazine derivatives and the yields thereof. Table 9 to be described later shows IR and NMR data of the obtained triazine derivatives.

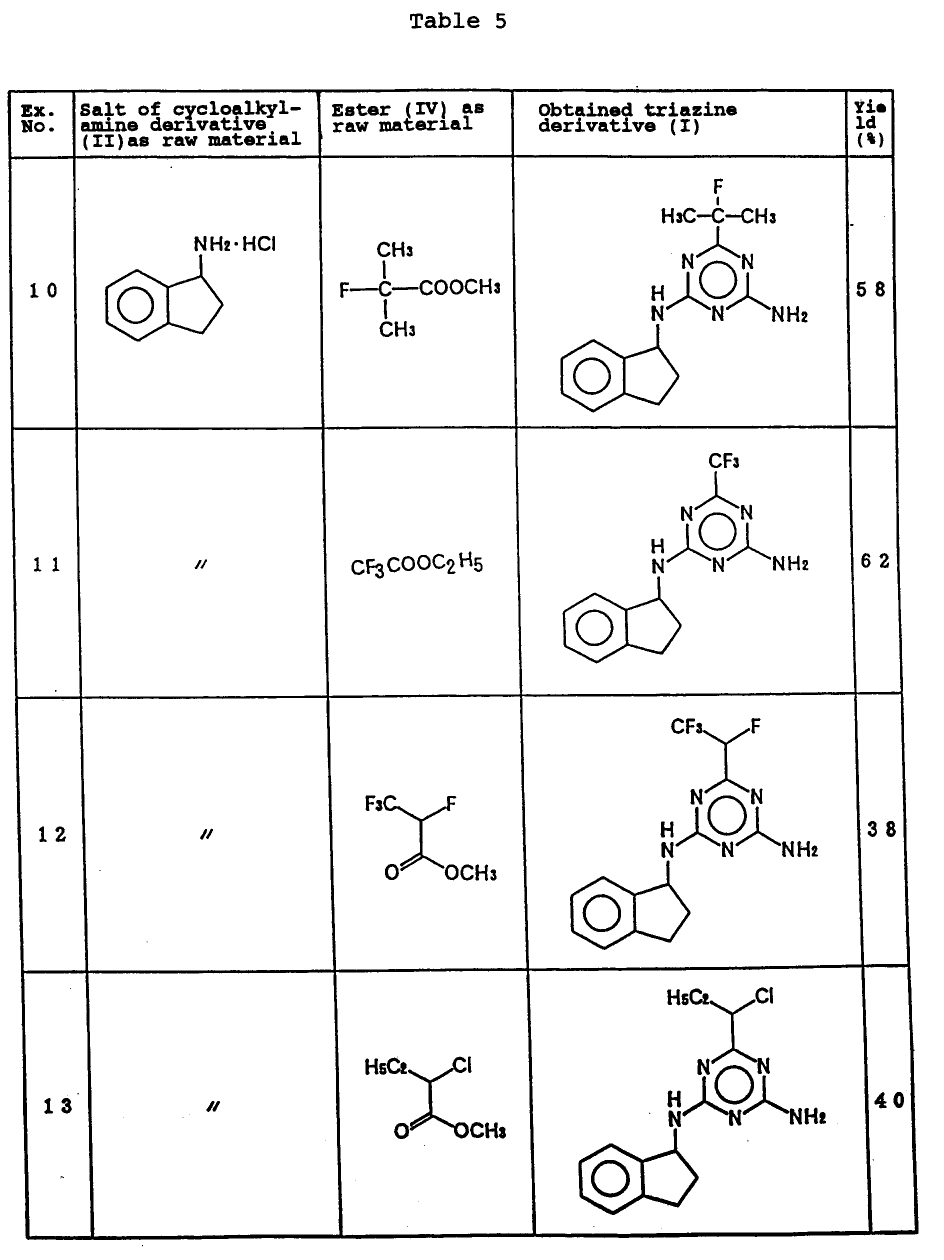

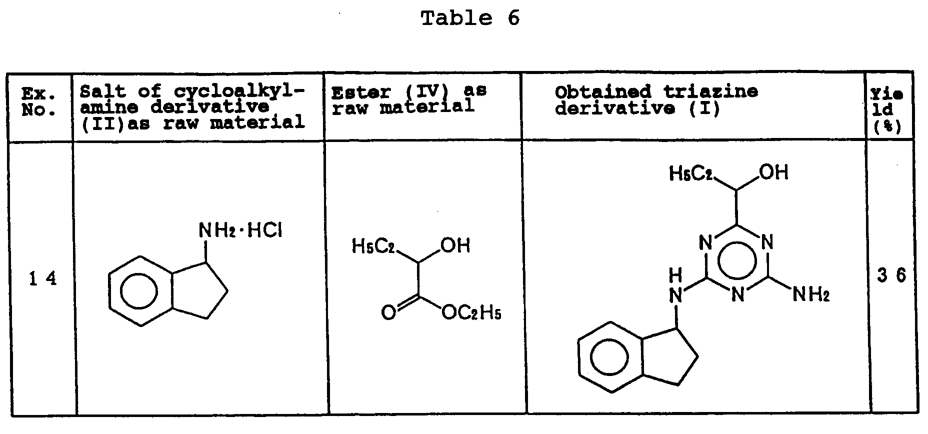

### (Examples 15 - 17)

Triazine derivatives as end products were obtained in the same manner as in Example 10 except that the 1-aminotetralin hydrochloride was replaced with salts of cycloalkylamine derivatives shown in Table 7. Table 7 to be described later show structural formulae of the salts of cycloalkylamine derivatives and the esters both of which are used as raw materials, the structural formulae of the obtained triazine derivatives and the yields thereof. Table 9 to be described later shows IR and NMR data of the obtained triazine derivatives.

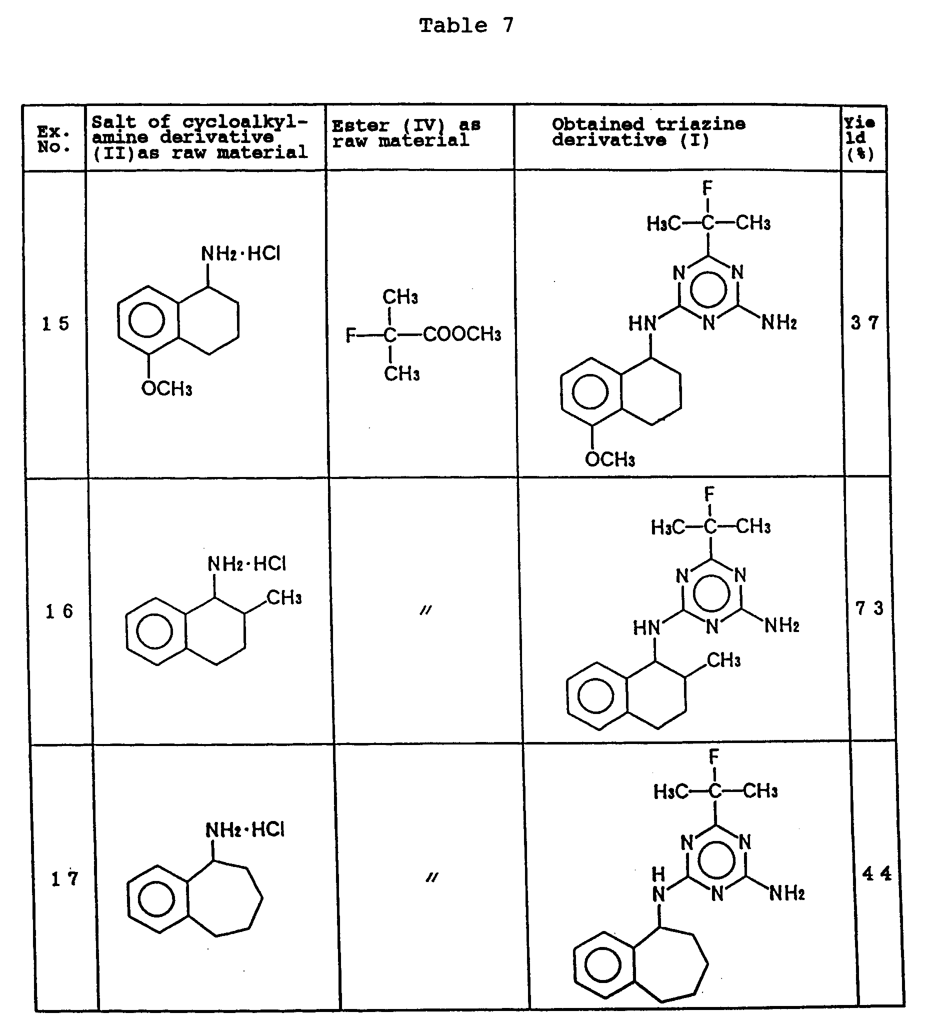

**Table 8**

| **Ex. No.** | **IR(cm**^{**-1**}**)****∗1** **s-triazine** | ^{**1**}**H-NMR****∗2** |
|---|---|---|
| 1 | 1550 | 1.63(3H, dd, J=7.8, 24.3Hz, CH₃-CHF-), 1.70-2.20(4H, m, Ar-CHCH₂CH₂), 2.70-2.95(2H, m, Ar-CH₂), 4.80-5.80(5H, m, CHF, NH₂, CH-NH), 7,00-7.45(4H, m, C₆H₄) |
| 2 | 1575 | 1.15-2.25(4H, m, Ar-CHCH₂CH₂), 1.65(6H, d, J=21.1Hz, 2Me), 2.50-3.00(2H, m, Ar-CH₂), 5.10-5.45(1H, m, NH), 5.45-5.85(1H, m, CH-NH), 5.85-6.60(2H, bs, NH₂), 6.90-7.50(4H, m, C₆H₄) |
| 3 | 1570 | 1.70-2.25(4H, m, Ar-CHCH₂CH₂), 2.65-2.95(2H, m, Ar-CH₂), 4.85-5.90(5H, m, CHF, NH₂, CH-NH), 7.00-7.45(4H, m, C₆H₄) |
| 4 | 1545 | 1.25(9H, s, t-Bu), 1.65-2.25(4H, m, Ar-CHCH₂CH₂), 2.60-2.95(2H, m, Ar-CH₂), 4.85-5.60(4H, m, NH₂, CH-NH), 6.95-7.50(4H, m, C₆H₄) |
| 5 | 1570 | 1.56(3H, dd, J=6.6, 24.7Hz, CH₃-CHF-), 1.60-2.05(4H, m, Ar-CHCH₂CH₂), 2.17(3H, s, Ar-CH₃), 2.23(3H, s, Ar-CH₃), 2.40-2.75(2H, m, Ar-CH₂), 4.60-6.50(5H, m, CHF, NH₂, CH-NH), 6.88(1H, s, C₆H), 6.96(1H, s, C₆H) |
| 6 | 1570 | 1.29(3H, d, J=7.3Hz, Ar-CHCH₃) 1.60(3H, dd, J=6.7, 24.6Hz, CH₃-CHF-), 1.55-2.40(4H, m, Ar-CHCH₂CH₂), 2.70-3.15(1H, m, Ar-CHCH₃), 4.75-6.50(5H, m, CHF, NH₂, CH-NH), 6.95-7.60(4H, m, C₆H₄) |
| 7 | 1580 | 1.50(3H, d, J=6.9Hz, CH₃-CHF), 1.60-2.10(4H, m, Ar-CHCH₂CH₂), 2.65-2.90(2H, m, Ar-CH₂), 3.78(3H, s, OCH₃), 4.80-5.80(5H, m, CHF, NH₂, CH-NH), 6.55-7.30(3H, m, C₆H₃) |
| 8 | 1570 | 1.45(3H, dd, J=6.8, 23.9Hz, CH₃-CHF-), 2.35-2.85(2H, m, Ar-CHCH₂), 2.70-3.15(2H, m, Ar-CH₂), 4.70-6.50(5H, m, CHF, NH₂, CH-NH), 7.05-7.50(4H, m, C₆H₄) |
| 9 | 1590 | 0.90-1.20(3H, m, CH₃), 1.40-2.40(3H, m, Ar-CHCHCH₂), 2.65-3.00(2H, m, Ar-CH₂), 4.85-5.25(1H, m, CH-NH), 5.25-6.20(3H, m, NH₂, NH), 7.00-7.45(4H, m, C₆H₄) |
| 10 | 1550 | 1.62(6H, d, J=22.8Hz, 2Me), 2.35-2.90(2H, m, Ar-CHCH₂), 2.75-3.10(2H, m, Ar-CH₂), 5.40-5.80(1H, m, CH-NH), 5.80-6.45(3H, m, NH₂, NH), 7.00-7.50(4H, m, C₆H₄) |

| | | |
|---|---|---|
| **∗1 Potassium bromide tablet method** | | |
| **∗2 Solvent: Deutero chloroform, Internal standard: Tetramethylsilane (TMS)** | | |

**Table 9**

| **Ex. No.** | **IR(cm**^{**-1**}**)****∗1** **s-triazine** | ^{**1**}**H-NMR****∗2** |
|---|---|---|
| 11 | 1590 | 2.40-2.85(2H, m, Ar-CHCH₂), 2.70-3.15(2H, m, Ar-CH₂), 5.35-5.90(2H, m, CH-NH), 5.85-6.45(2H, m, NH₂), 7.05-7.50(4H, m, C₆H₄) |
| 12 | 1590 | 2.50-3.00(2H, m, Ar-CHCH₂), 2.80-3.20(2H, m, Ar-CH₂), 4.90-6.00(5H, m, CHF, NH₂, CH-NH), 7.10-7.60(4H, m, C₆H₄) |
| 13 | 1540 | 1.03(3H, t, J=7.6Hz, CH₃), 1.80-2.40(2H, m, CH₃CH₂), 2.40-2.80(2H, m, Ar-CHCH₂), 2.75-3.10(2H, m, Ar-CH₂), 4.44(1H, q, J=7.6Hz, CHCl), 5.25-5.95(4H, m, CH-NH, NH₂), 7.05-7.50(4H, m, C₆H₄) |
| 14 | 1560 | 1.00(3H, t, J=6.8Hz, CH₃), 1.50-2.30(2H, m, CH₃CH₂), 2.40-2.85(2H, m, Ar-CHCH₂), 2.75-3.15(2H, m, Ar-CH₂), 4.15-4.50(1H, m, CHOH), 5.00-5.35(2H, m, CH-NH), 5.35-5.80(2H, m, NH₂), 7.10-7.40(4H, m, C₆H₄) |
| 15 | 1590 | 1.61(6H, d, J=22.0Hz, CH₃-CF-CH₃), 1.60-2.15(4H, m, Ar-CHCH₂CH₂), 2.60-2.90(2H, m, Ar-CH₂), 3.75(3H, s, OCH₃), 5.00-5.40(2H, m, CH-NH), 5.35-5.65(2H, m, NH₂), 6.50-7.35(3H, m, C₆H₃) |
| 16 | 1570 | 0.85-1.25(3H, m, CH₃), 1.63(6H, d, J=22.0Hz, CH₃-CF-CH₃), 1.40-2.30(3H, m, CHCH₂), 2.65-3.05(2H, m, Ar-CH₂), 4.80-5.20(1H, m, CH-NH), 5.25-6.30(3H, m, NH, NH₂), 6.90-7.40(4H, m, C₆H₄), |
| 17 | 1570 | 1.20-2.10(6H, m, Ar-CHCH₂CH₂CH₂), 1.62(6H, d, J=22.2Hz, CH₃-CF-CH₃), 2.65-3.10(2H, m, Ar-CH₂), 5.00-5.40(1H, m, NH), 5.60-6.60(3H, m, NH₂, CH-NH), 6.85-7.40(4H, m, C₆H₄) |

| | | |
|---|---|---|
| **∗1 Potassium bromide tablet method** | | |
| **∗2 Solvent: Deutero chloroform, Internal standard: Tetramethylsilane (TMS)** | | |

### (Examples 18 - 31)

Triazine derivatives as end products were obtained from salts of cycloalkylamine derivatives and esters shown in Tables 10 to 14 in the same manner as in Examples 1 to 17. Tables 10 to 14 to be described later show the structural formulae of the salts of cycloalkylamine derivatives and the esters both of which are used as raw materials, the structural formulae of the obtained triazine derivatives and the yields thereof.

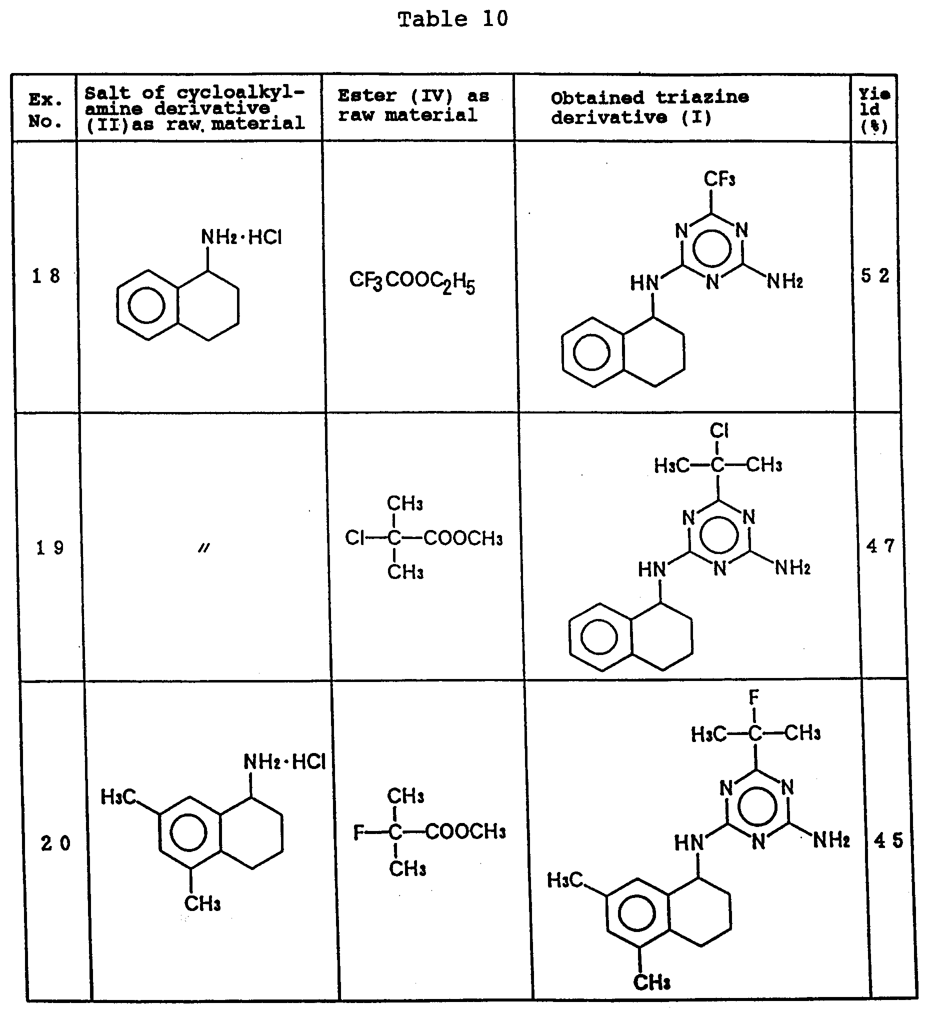

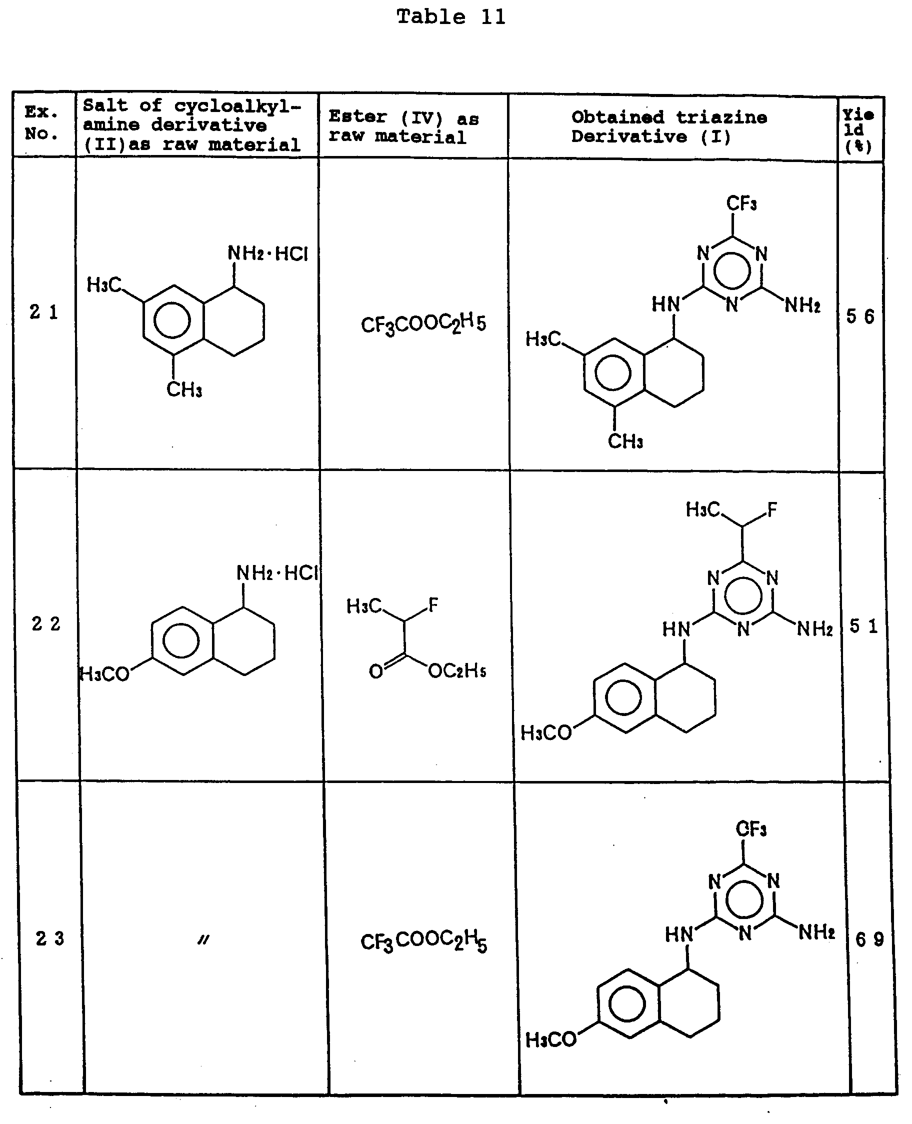

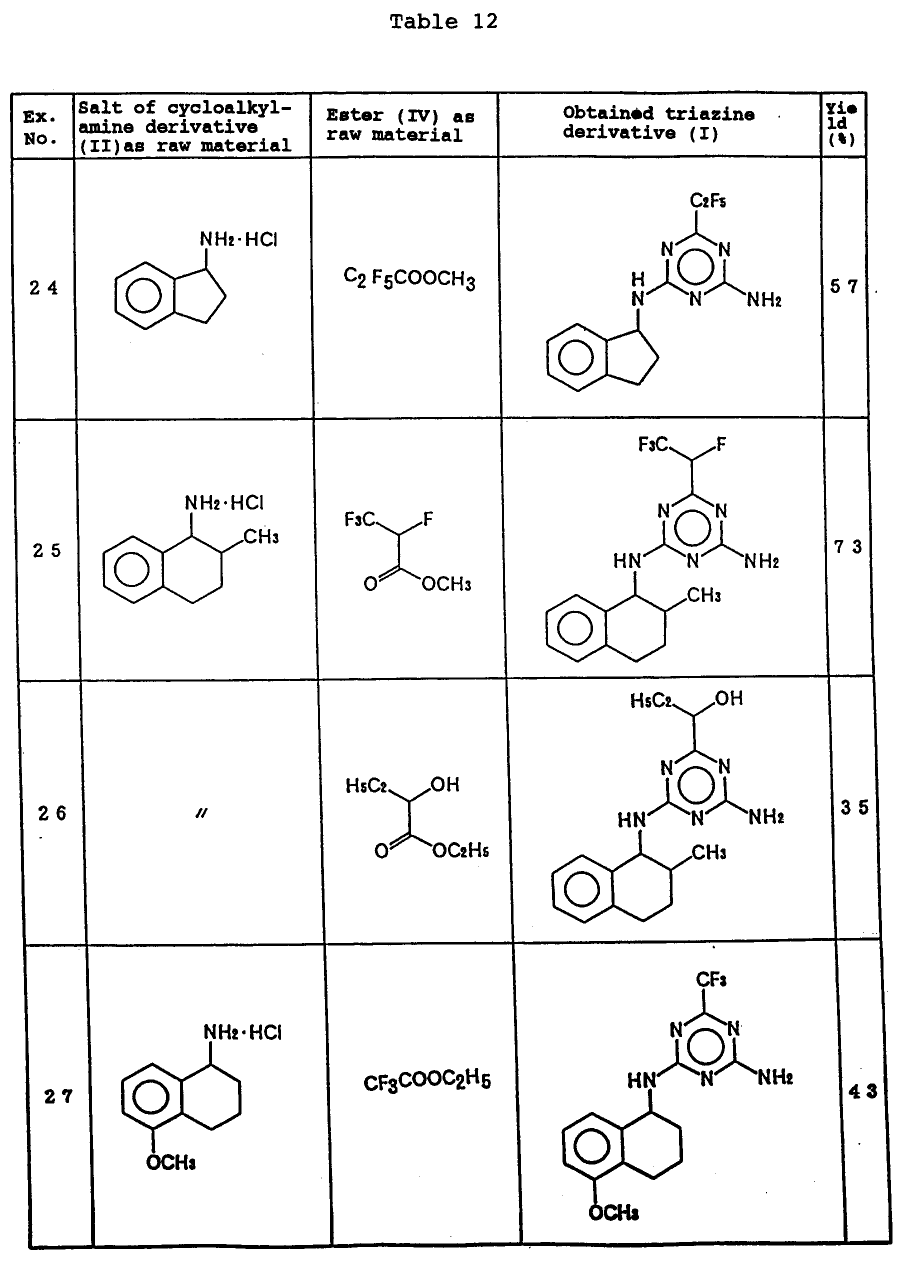

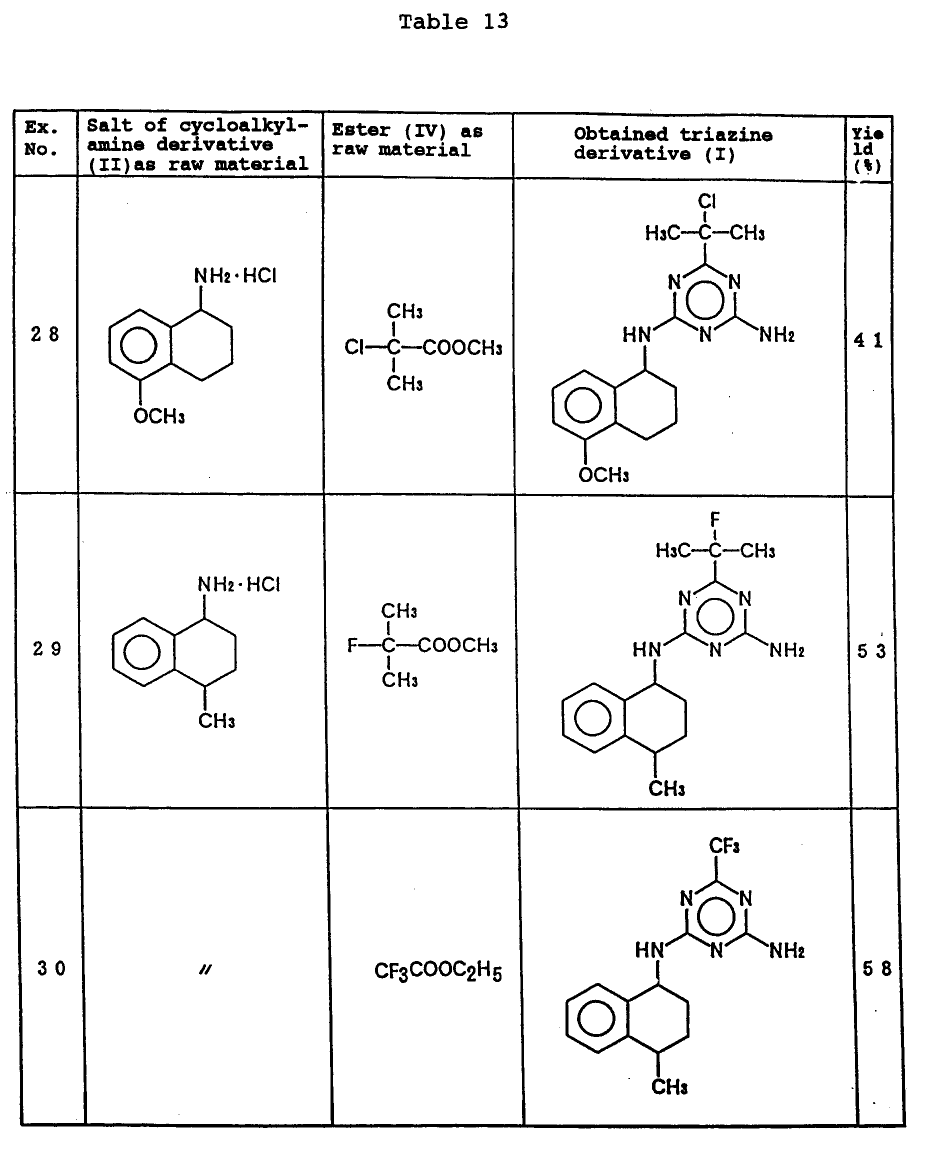

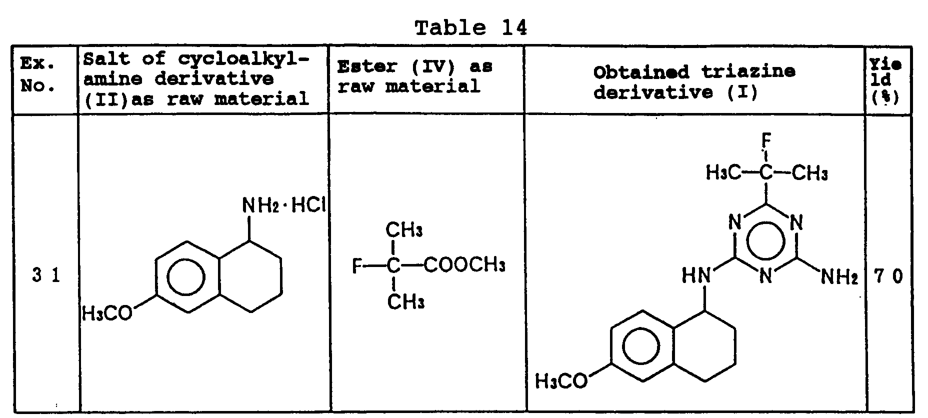

### (Example 32)

0.98 Gram (5.2 mmol) of 6-fluoro-4-chromanylamine hydrochloride, 0.44 g (5.2 mmol) of cyanoguanidine and 20 ml of n-decane were placed in a reactor, and stirred at 135°C for 6 hours. After completion of the reaction, the reaction mixture was cooled, a formed precipitate was recovered by filtration and washed with 5 ml of n-hexane three times, and the solvent contained in the obtained precipitate was removed under reduced pressure to give 1 g of a solid. This solid was dissolved in 25 ml of absolute methanol. To the resultant solution was added 1.9 g (10 mmol) of a 28 wt% sodium methoxide/methanol solution at room temperature. Further, 1.2 g (10 mmol) of methyl α-fluoroisobutyrate was dropwise added thereto, and the mixture was stirred at room temperature for 12 hours. After completion of the reaction, the reaction mixture was poured into 100 ml of water, and extraction was carried out with 50 ml of ethyl acetate three times. An obtained ethyl acetate layer was dried over anhydrous sodium sulfate, and the ethyl acetate was distilled off under reduced pressure. The resultant residue was purified by silica gel column chromatography (developer solution: hexane/ethyl acetate = 1/1 (volume ratio)) to give 0.68 g (yield 41 %) of 2-amino-4-(α-fluoro-α-methylethyl)-6-(6-fluoro-4-chromanyl)amino- s-triazine as an end product in the form of a white crystal. Table 15 shows the structure and the yield of the obtained product, and Table 33 shows IR and NMR data thereof.

### (Examples 33 - 38)

The same procedures as those in Example 32 were repeated except that the methyl α-fluoroisobutyrate used in Example 32 was replaced with esters shown in Table 15 or 16. Table 15 or 16 shows the structures and the yields of obtained products, and Table 33 shows IR and NMR data of the obtained products.

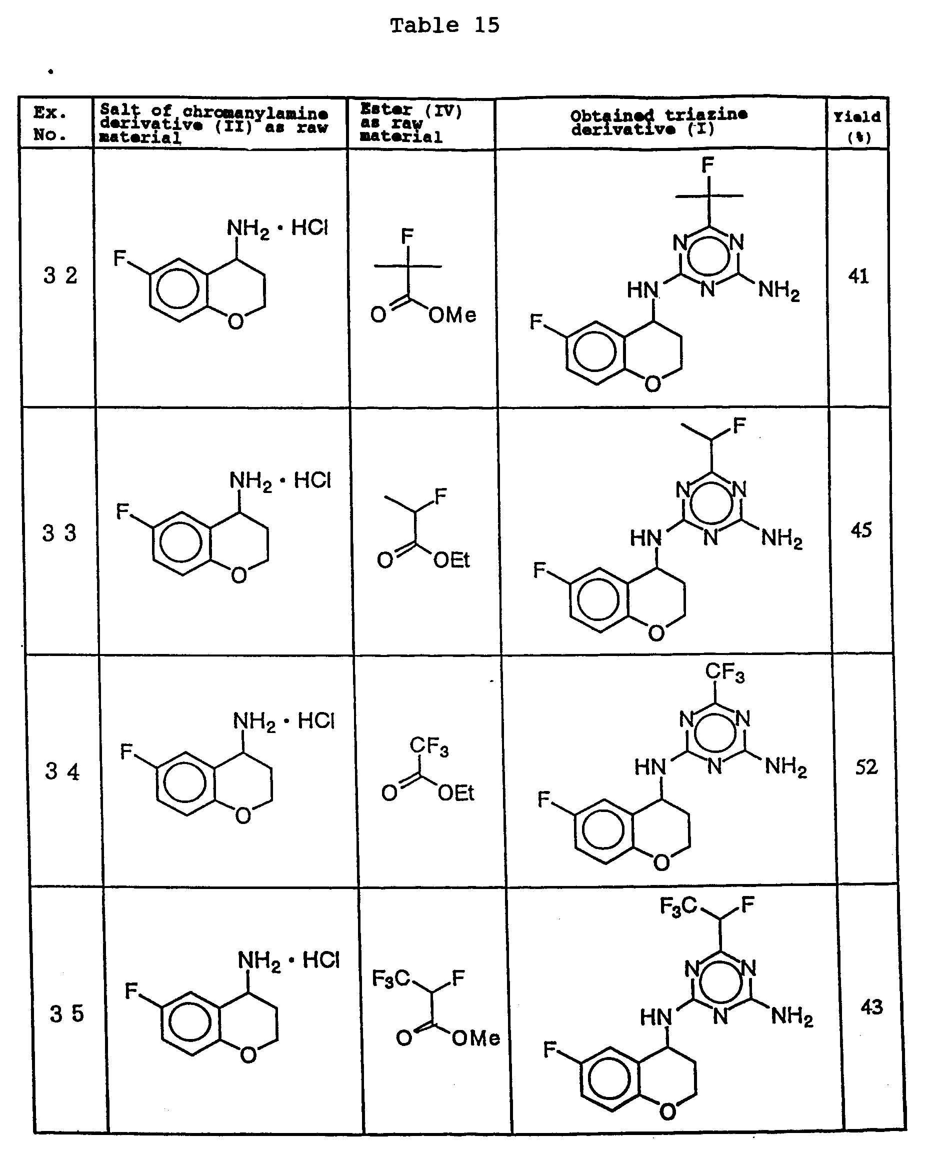

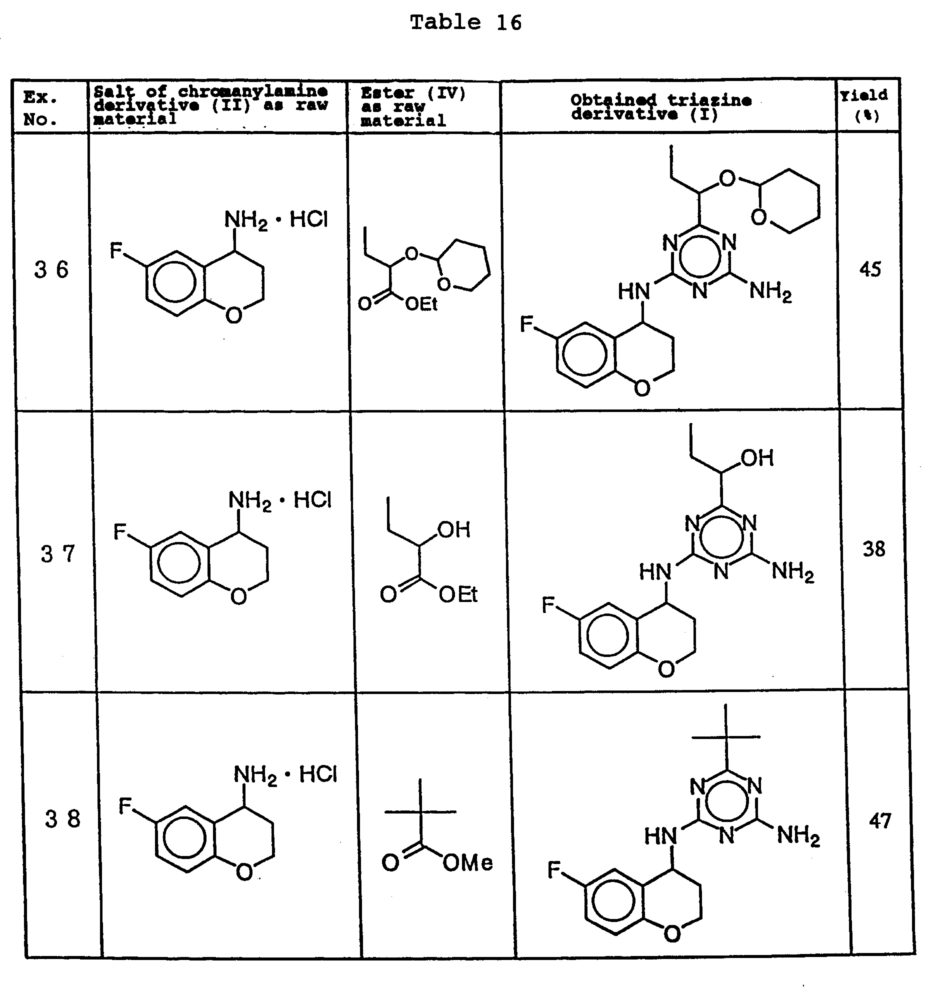

### (Examples 39 - 75)

The same procedures as those in Example 32 were repeated except that the 6-fluoro-4-chromanylamine hydrochloride used in Example 32 was replaced with 4-chromanylamine hydrochlorides shown in Tables 17 to 25. Tables 17 to 25 show the structures and the yields of obtained products, and Tables 33 to 37 show IR and NMR data of the obtained products.

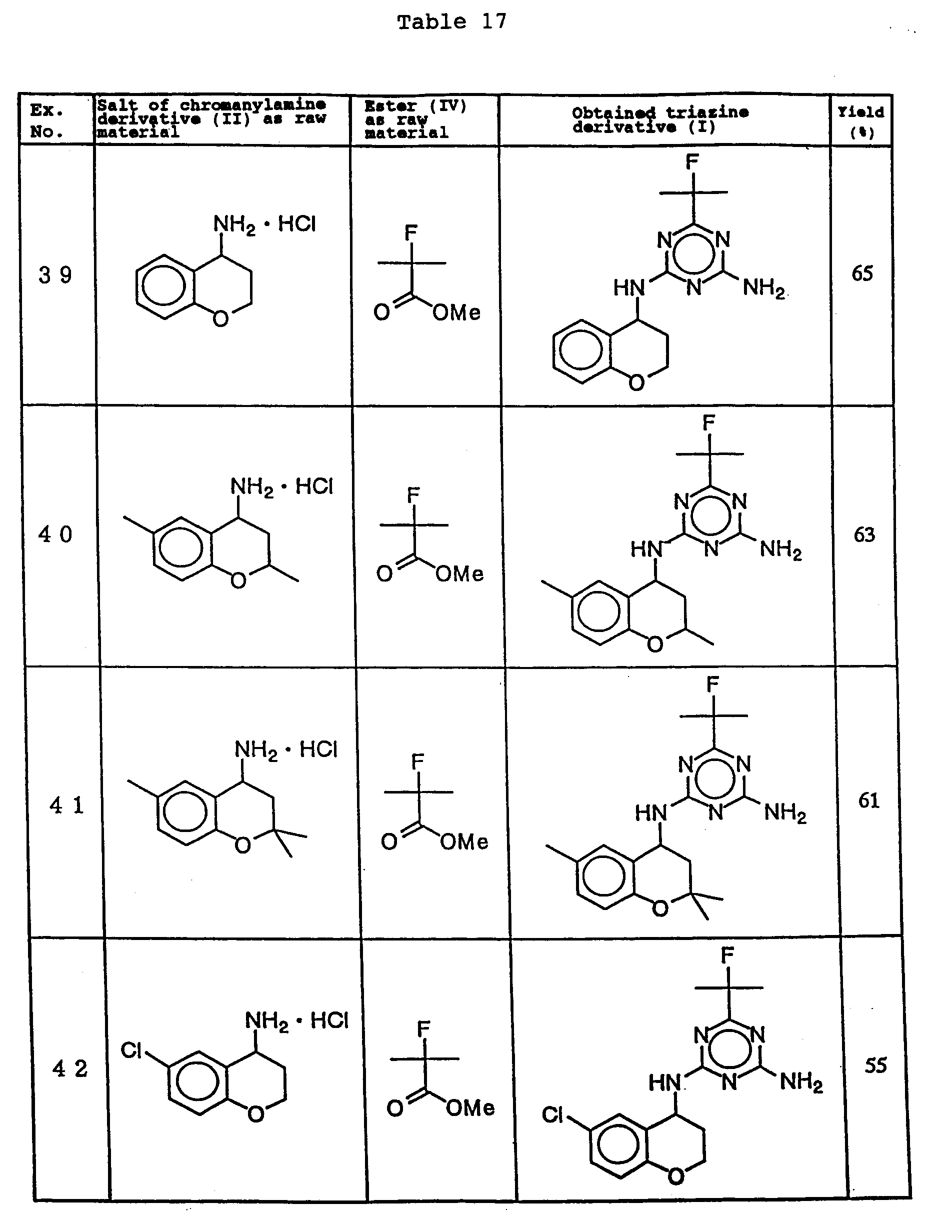

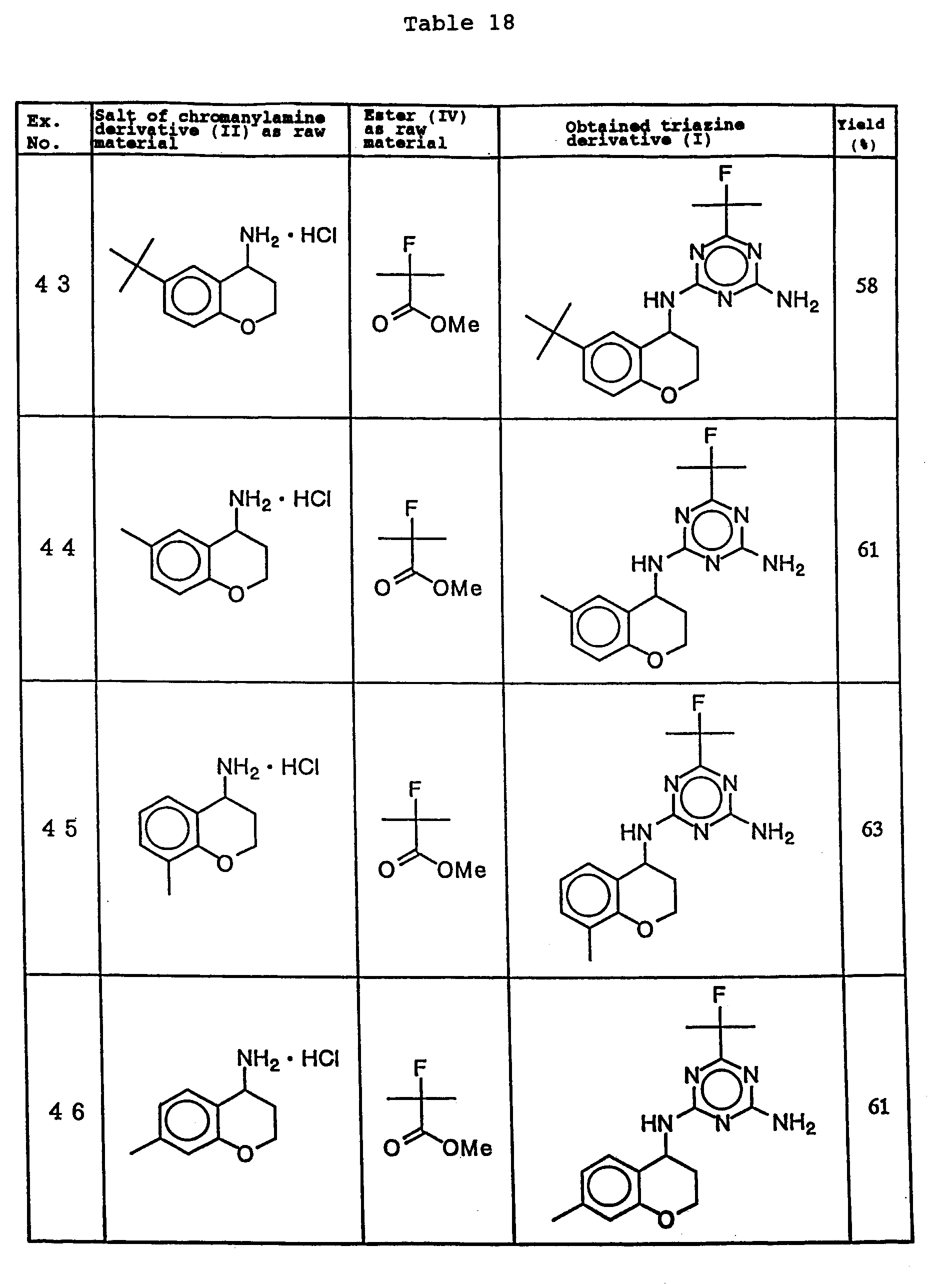

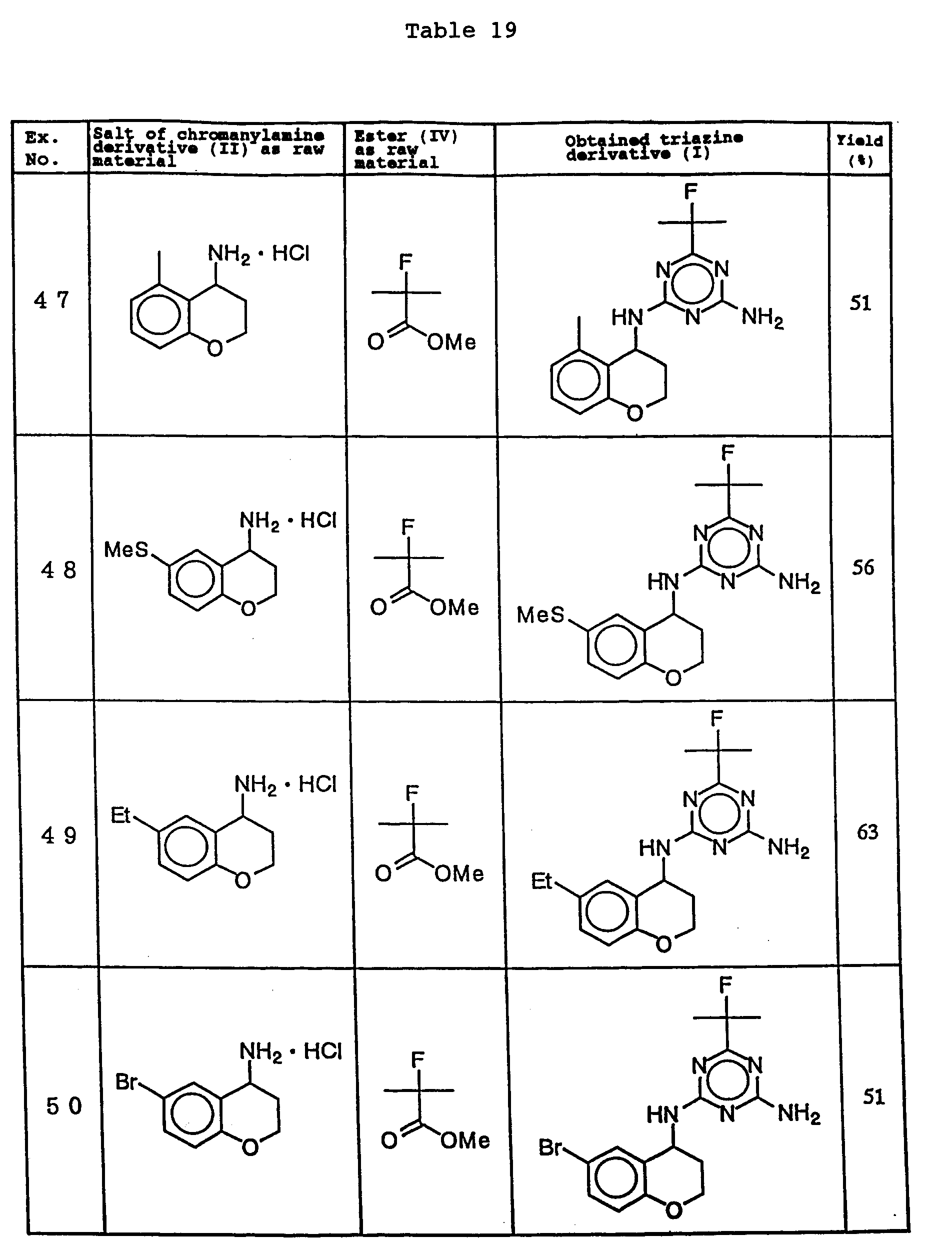

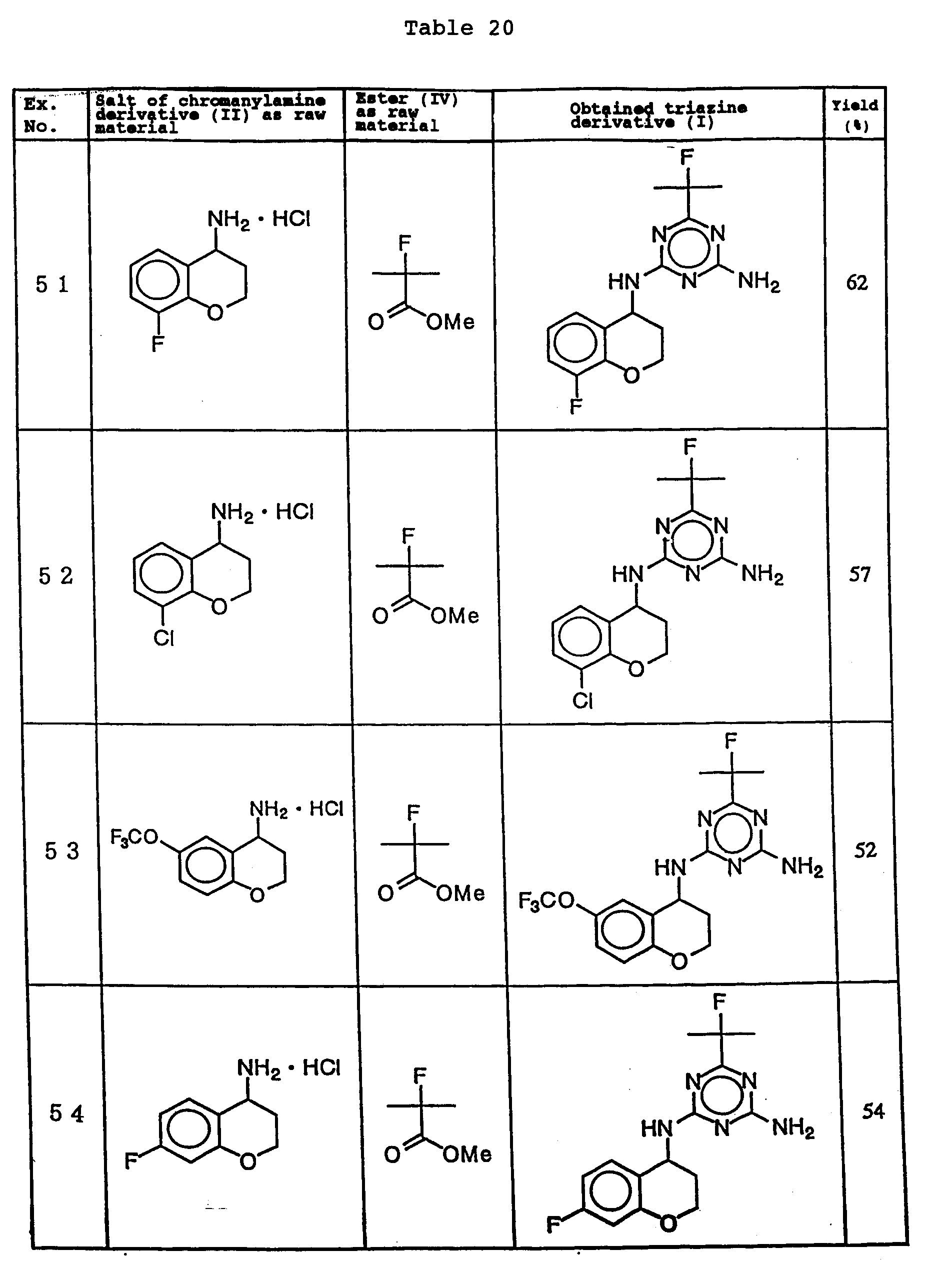

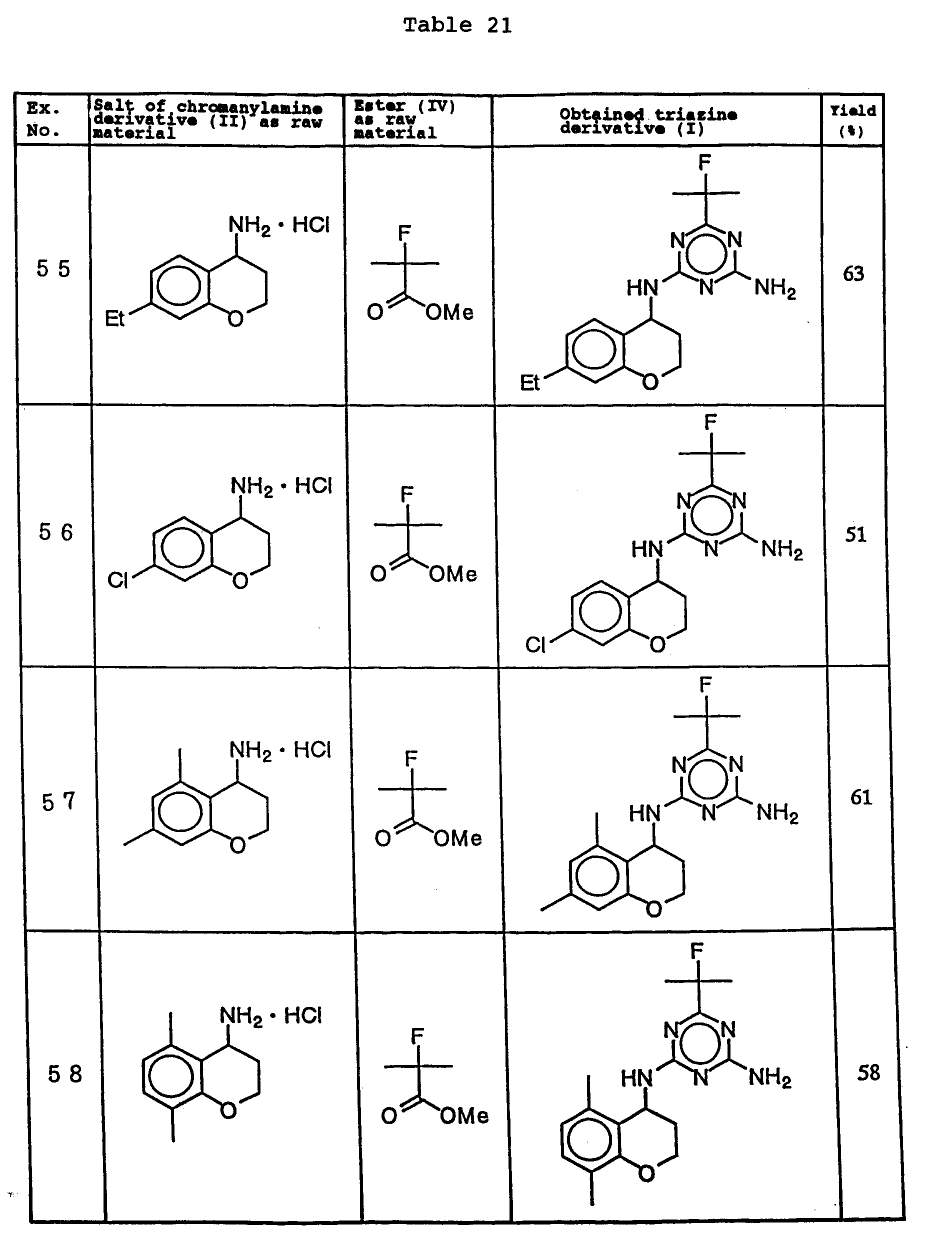

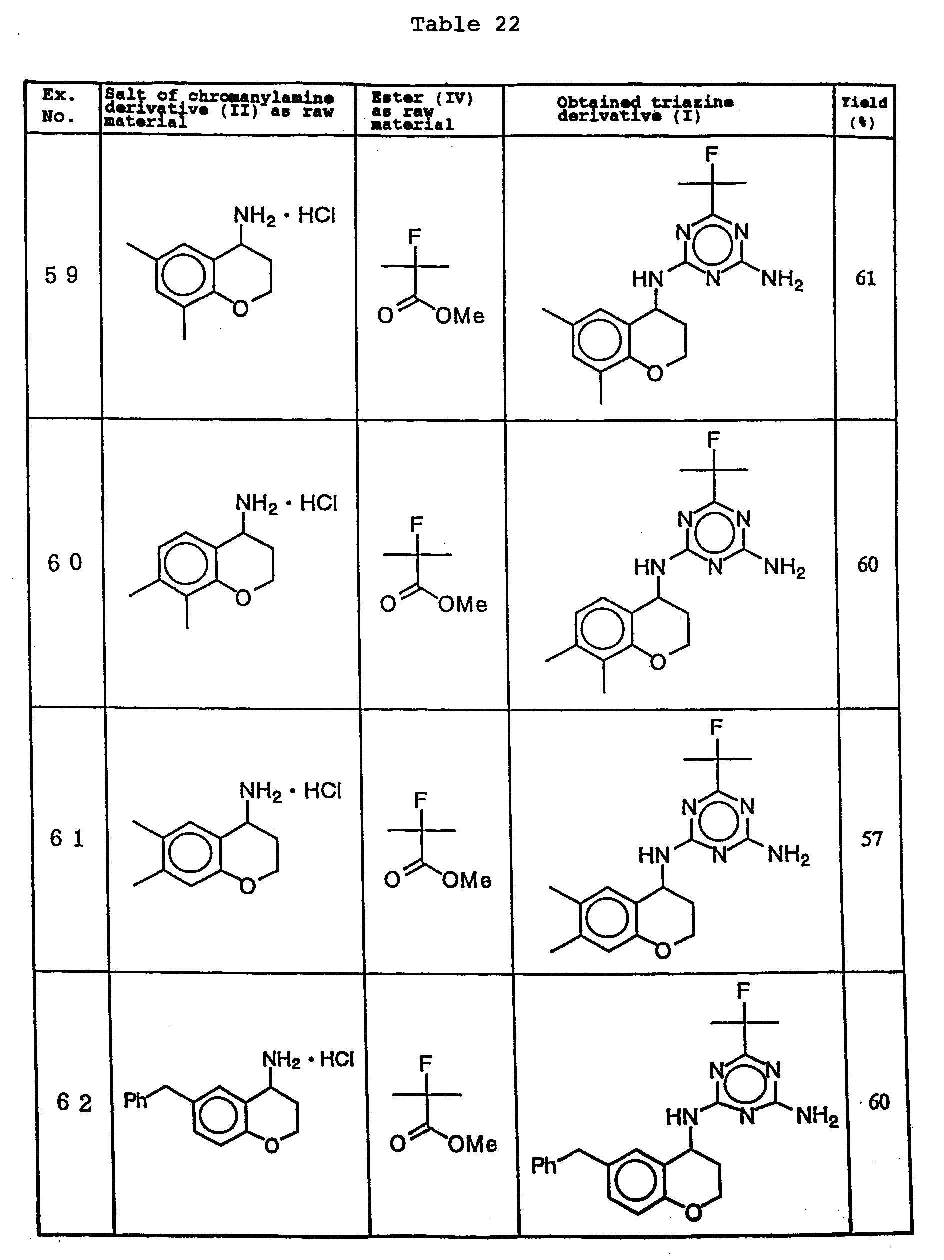

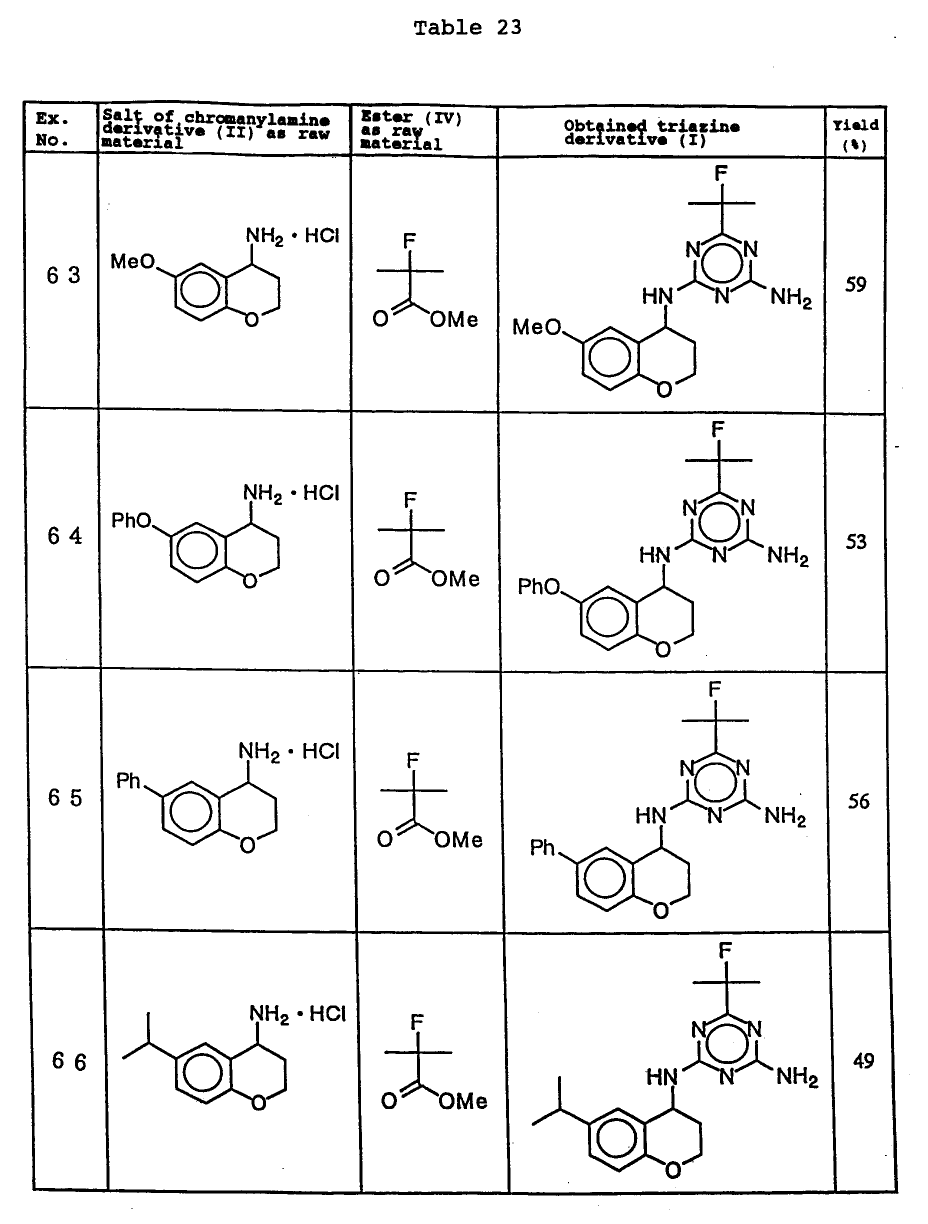

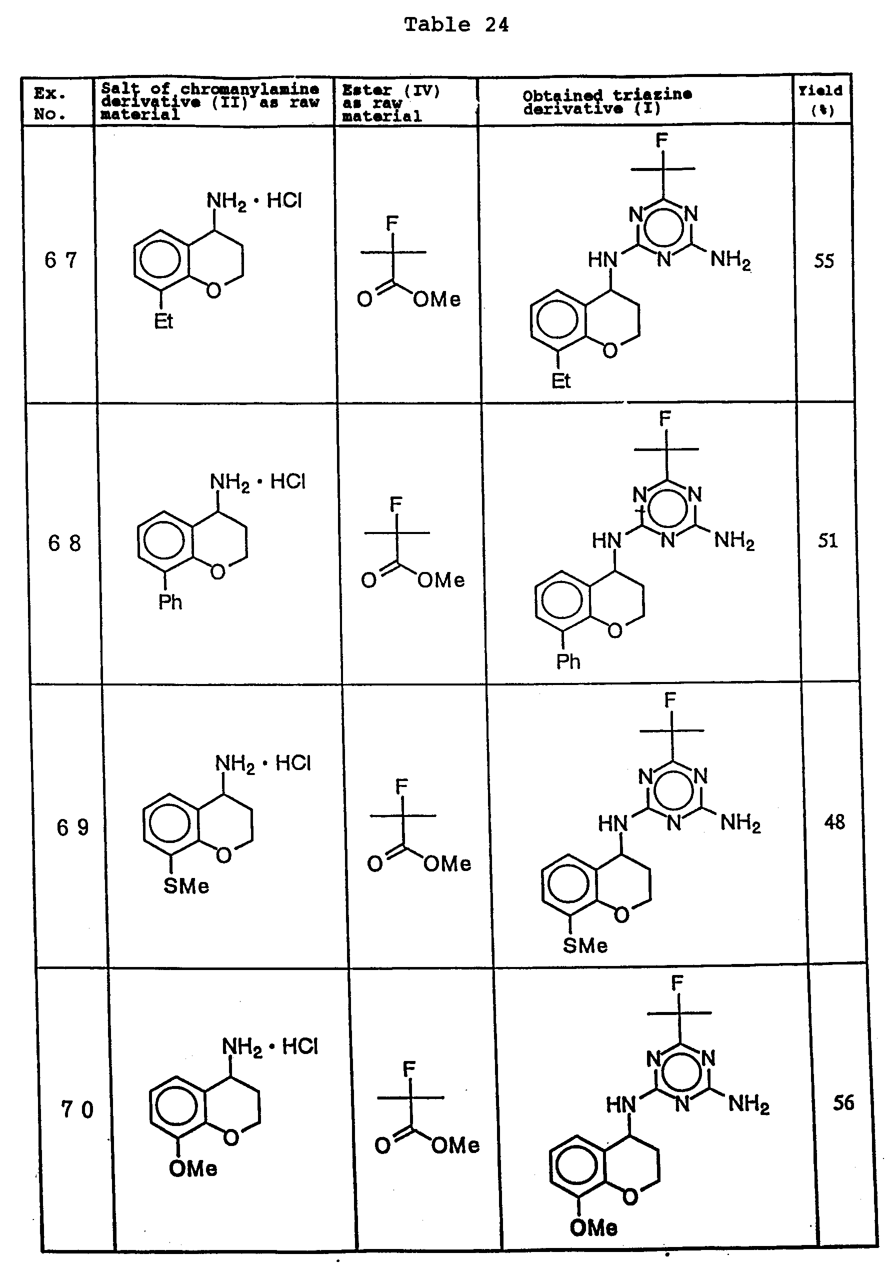

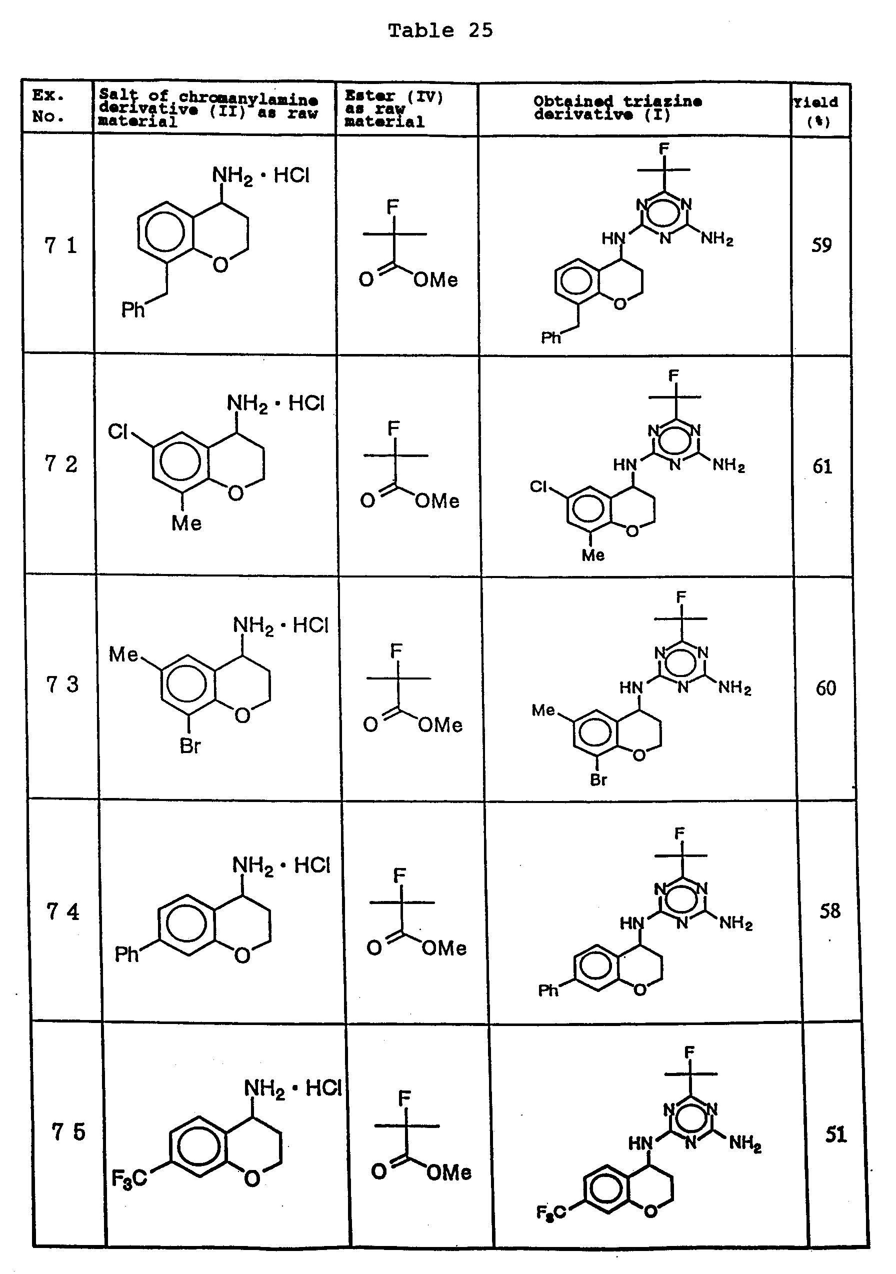

### (Examples 76 - 97)

The same procedures as those in Example 32 were repeated except that the 6-fluoro-4-chromanylamine hydrochloride and the methyl α-fluoroisobutyrate used in Example 32 were replaced with 4-chromanylamine hydrochlorides and esters shown in Tables 26 to 31. Tables 26 to 31 show the structures and the yields of obtained products, and Tables 37 to 40 show IR and NMR data of the obtained products.

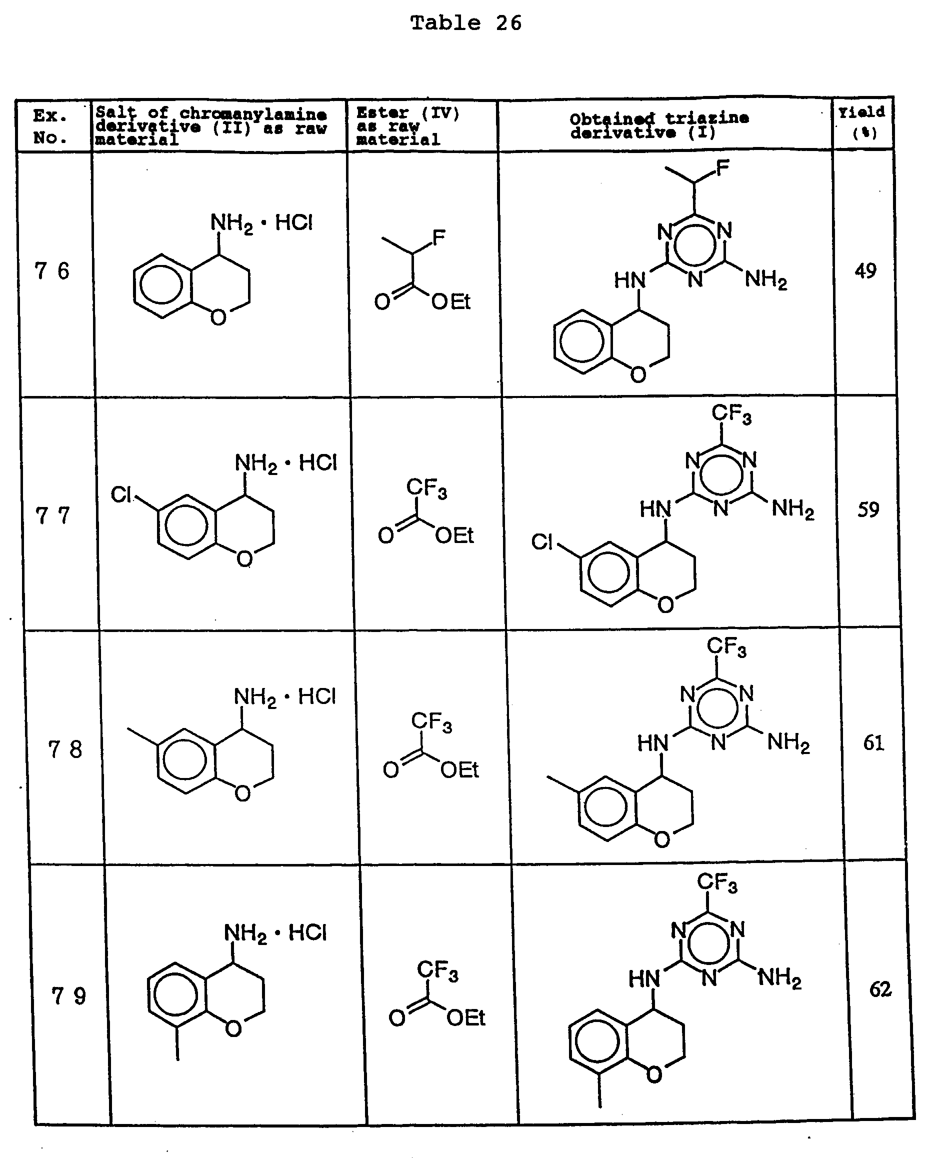

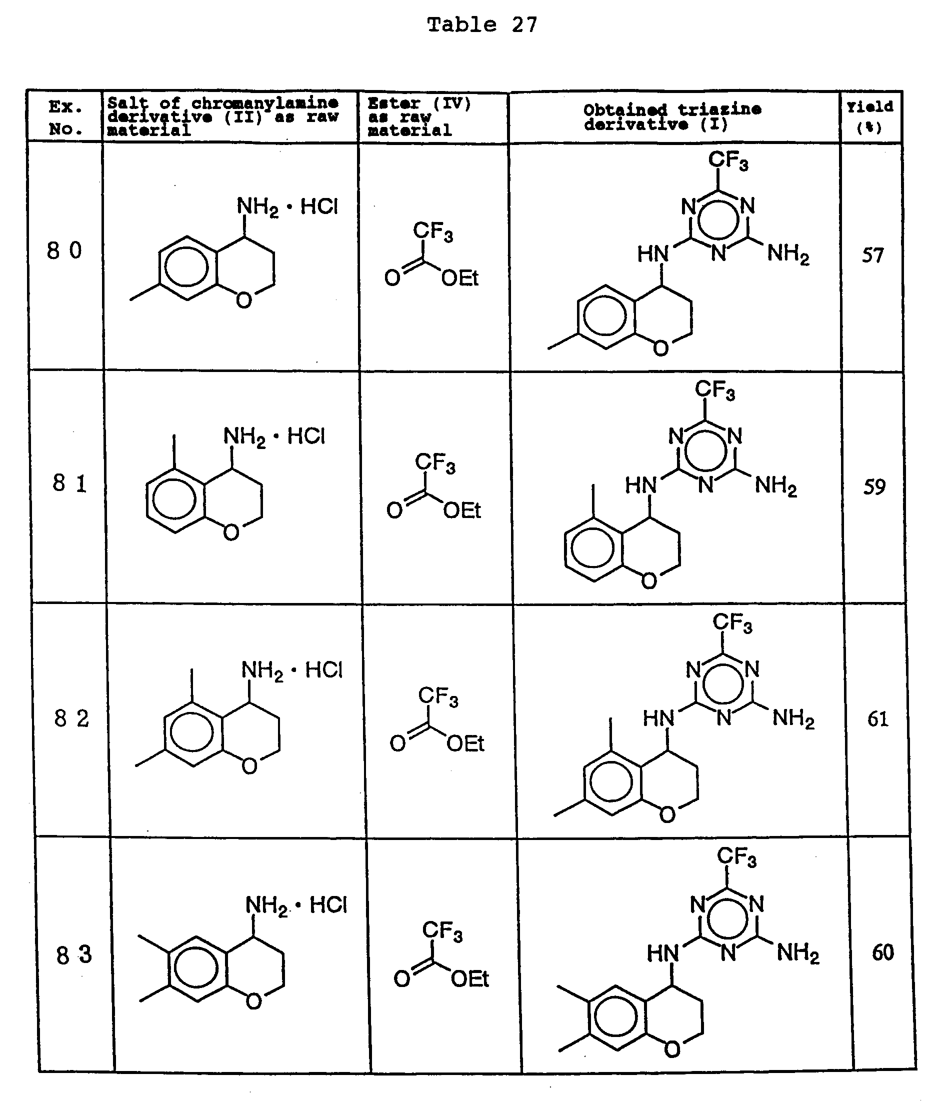

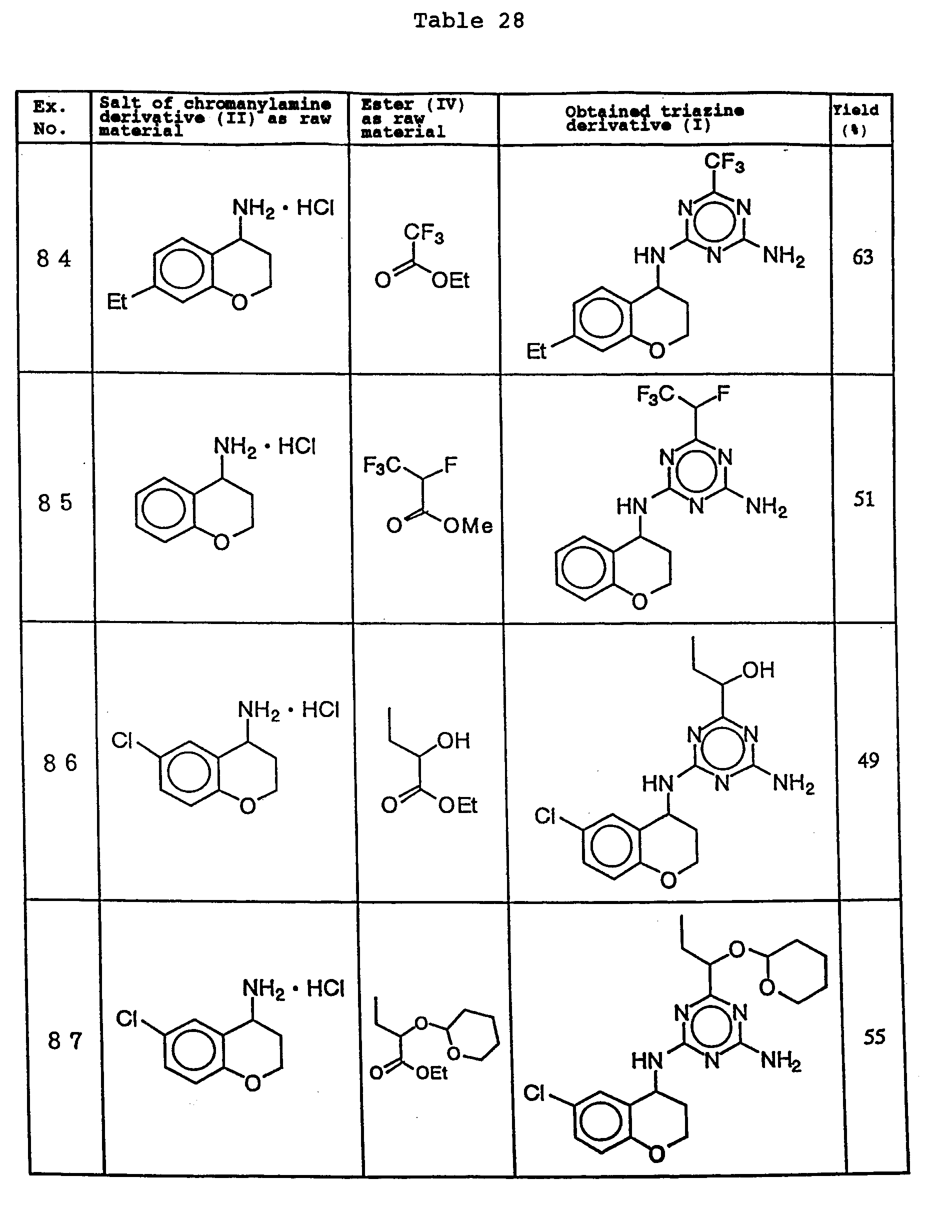

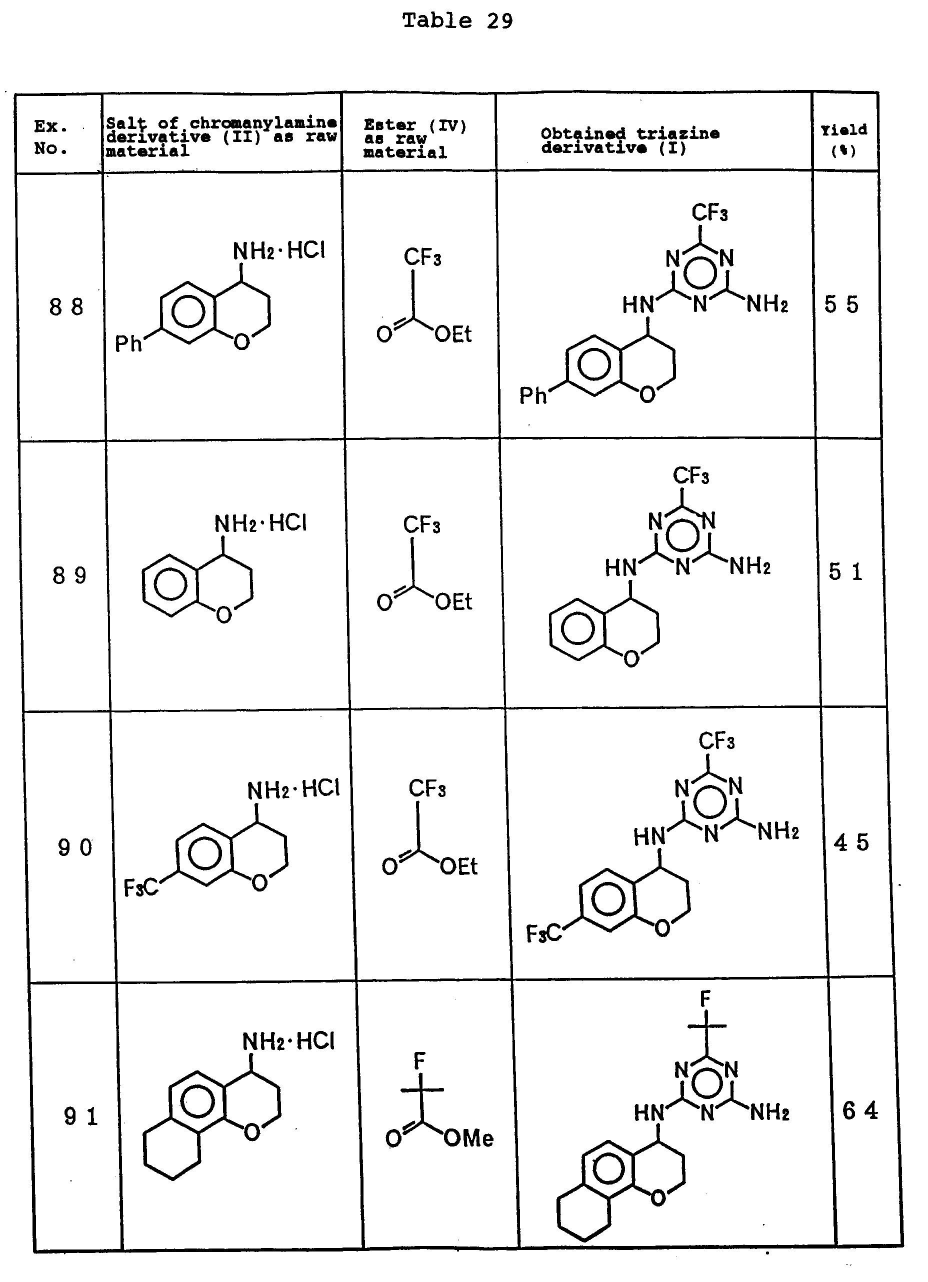

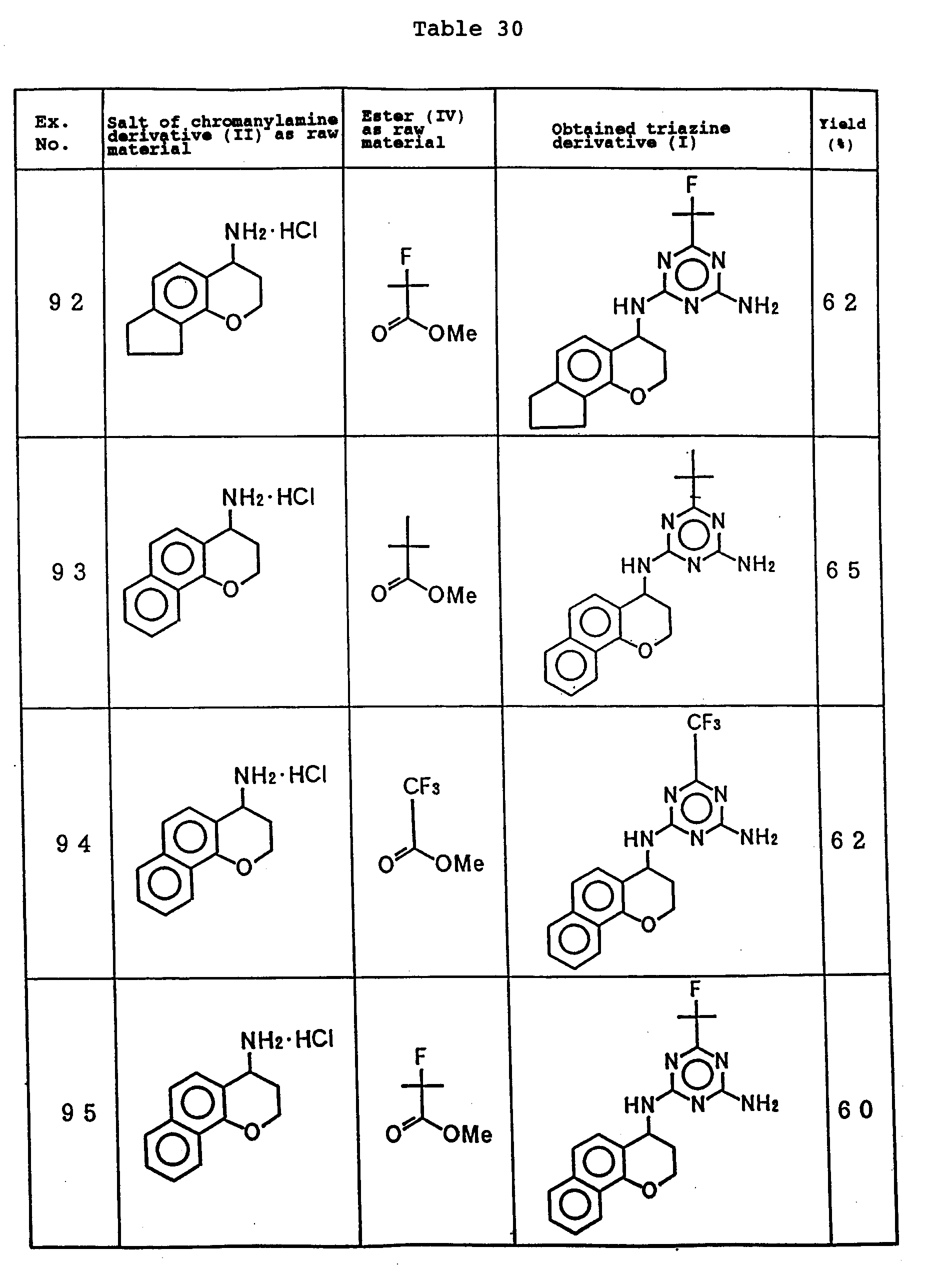

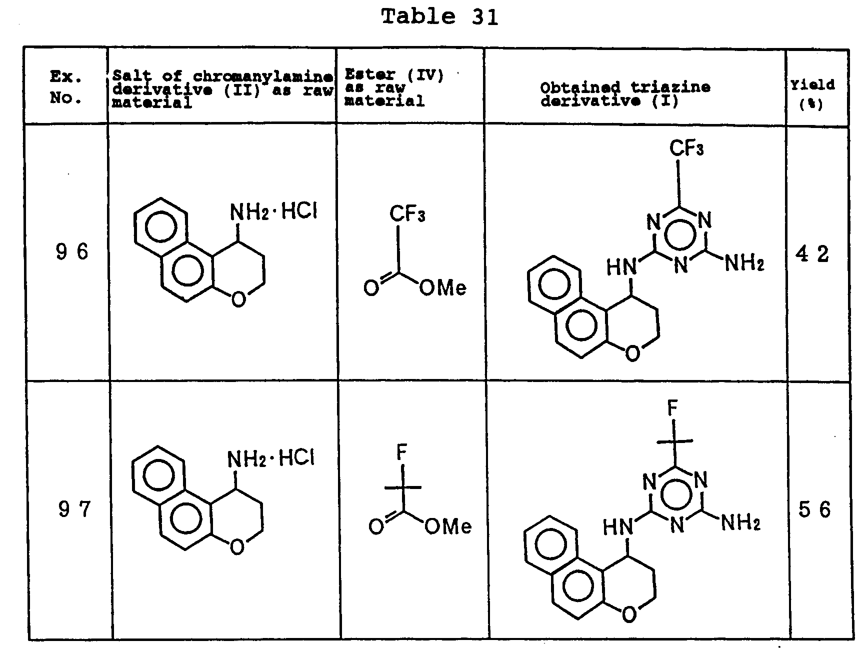

### (Example 98)

1 Gram (2.87 mmol) of the 2-amino-4-(α-fluoro-α-methylethyl)-6-(6-methylthio-4-chromanyl)amino-s-triazine obtained in Example 48 was dissolved in 15 ml of ethyl acetate, and to this mixture was added 1.1 g (6.38 mmol) of m-chloroperbenzoic acid at room temperature. The reaction mixture was stirred at room temperature for 12 hours and then washed with 10 ml of a 5 wt% sodium sulfite aqueous solution, and an ethyl acetate layer was washed with 10 ml of water twice. The ethyl acetate layer was dried over anhydrous sodium sulfate, and then the ethyl acetate was distilled off under reduced pressure. The resultant residue was purified by silica gel column chromatography (developer solvent: n-hexane/ethyl acetate = 1/1 (volume ratio)) to give 0.89 g (yield 82 %) of 2-amino-4-(α-fluoro-α-methylethyl)-6-(6-methanesulfonyl-4-chromanyl)amino-s-triazine as an end product. Table 32 shows the structure and the yield of the obtained product, and Table 40 shows the IR and NMR data of the product.

### (Example 99)

2-Amino-4-(α-fluoro-α-methylethyl)-6-(8-methanesulfonyl-4-chromanyl)amino-s-triazine was obtained in the same manner as in Example 98 except that the 2-amino-4-(α-fluoro-α-methylethyl)-6-(6-methylthio-4-chromanyl)amino-s-triazine used in Example 98 was replaced with 2-amino-4-(α-fluoro-α-methylethyl)-6-(8-methylthio-4-chromanyl)amino-s-triazine. Table 32 shows the structure and the yield of the obtained product, and Table 40 shows the IR and NMR data of the product.

### (Example 100)

0.9 Gram (10.0 mmol) of cuprous cyanide was added to a solution of 3.2 g (8.4 mmol) of the 2-amino-4-(6-bromo-4-chromanyl)amino-6-(α-fluoro-α-methylethyl)-s-triazine obtained in Example 50 in 3 ml of DMF, and the mixture was refluxed under heat for 4 hours. To the reaction mixture was added a saturated ammonium choride aqueous solution, the mixture was filtered, and a solid substance was washed with ethyl acetate. A filtrate and a wash liquid were combined, an organic layer was extracted with ethyl acetate, and an extract was dried over anhydrous sodium sulfate. Then, the solvent was distilled off under reduced pressure. The resultant residue was purified by silica gel column chromatography [silica gel: 150 g, developer solvent: n-hexane/ethyl acetate = 1/1 (volume ratio)] to give 2.4 g (yield 87 %) of 2-amino-4-(6-cyano-4-chromanyl)amino-6-(α-fluoro-α-methylethyl)-s-triazine as an end product. Table 32 shows the structure and the yield of the obtained product, and Table 40 shows the IR and NMR data of the product.

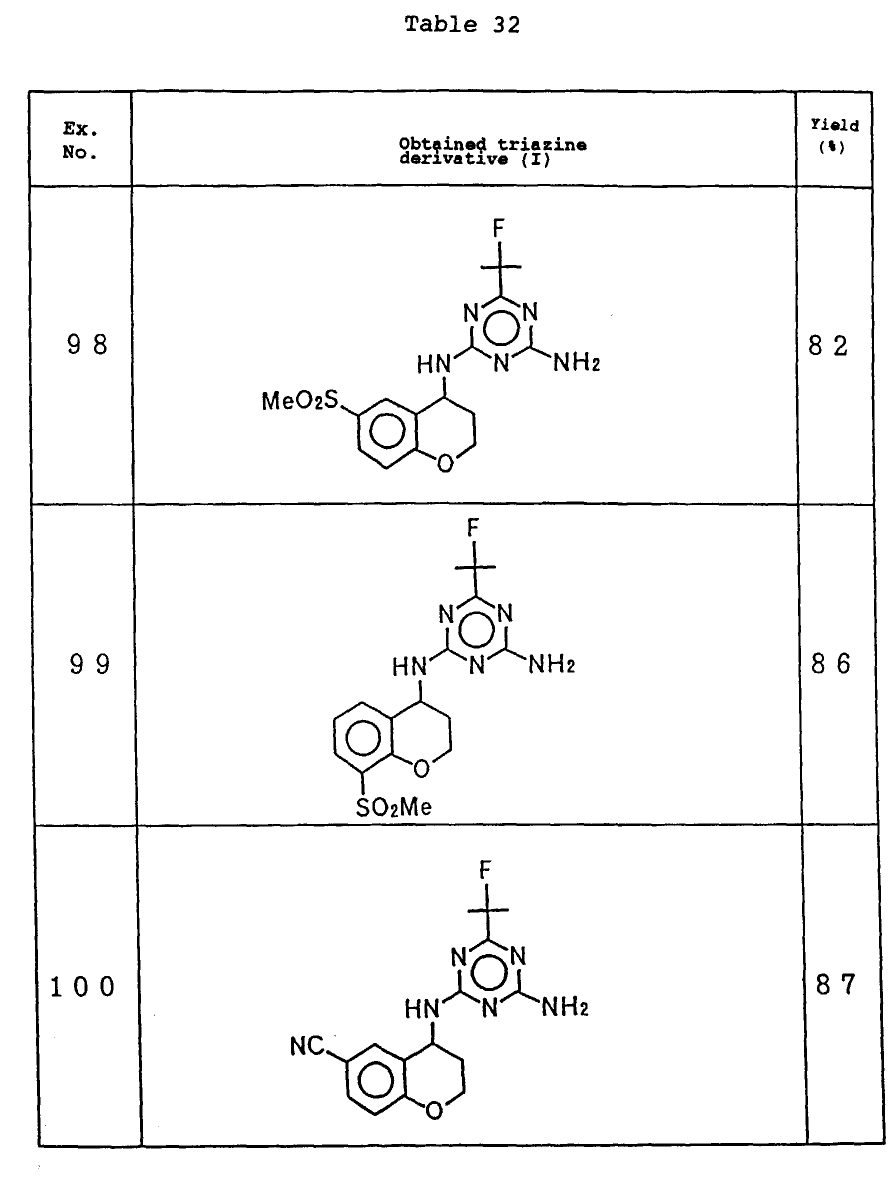

**Table 33**

| **IR◆NMR data** | | |
|---|---|---|
| **Example No.** | **IR(cm**^{**-1**}**)****∗1** **s-triazine** | ^{**1**}**H-NMR*******^{**2**} |
| 32 | 1555 | 1.65(6H, d, J=21.9Hz, CH₃-CF-CH₃), 2.00-2.30(2H, m, OCH₂CH₂), 4.10-4.30(2H, m, OCH₂), 5.10-5.40(1H, m, CH-N), 5.40-6.00(3H, m, NH, NH₂), 6.70-7.10(3H, m, Ar) |
| 33 | 1570 | 1.59(3H, dd, J=24.6, 6.7Hz, CH₃), 2.00-2.30(2H, m, OCH₂CH₂), 4.10-4.30(2H, m, OCH₂), 4.70-6.60(5H, m, CH-NH, NH₂, CHF), 6.70-7.10(3H, m, Ar) |
| 34 | 1580 | 2.05-2.35(2H, m, OCH₂CH₂), 4.10-4.30(2H, m, OCH₂), 5.00-6.30(4H, m, CH-NH, NH₂), 6.70-7.10(3H, m, Ar) |
| 35 | 1580 | 2.00-2.35(2H, m, OCH₂CH₂), 4.10-4.30(2H, m, OCH₂), 4.90-5.80(5H, m, CH-NH, NH₂, CHF), 6.70-7.10(3H, m, Ar) |
| 36 | 1570 | 0.85-1.15(3H, m, CH₃), 1.35-2.40(10H, m, OCH₂CH₂, CH₂-CH₃,THP), 3.20-4.40(5H, m, OCH₂, OCH, THP) 4.60-5.80(5H, m, CH-NH, NH₂, THP), 6.70-7.10(3H, m, Ar) |
| 37 | 1570 | 0.98(3H, t, J=7.5Hz, CH₃), 1.67(1H, s, OH). 1.80-2.30(4H, m, OCH₂CH₂, CH₂-CH₃), 4.15-4.45(3H, m, OCH₂, CH-OH), 5.00-5.60(4H, m, CH-NH, NH₂), 6.70-7.10(3H, m, Ar) |
| 38 | 1570 | 1.26(9H, s, t-Bu), 2.05-2.25(2H, m, OCH₂CH₂), 4.10-4.30(2H, m, OCH₂), 5.00-5.70(4H, m, CH-NH, NH₂), 6.70-7.10(3H, m, Ar) |
| 39 | 1535 | 1.57(6H, d, J=21.3Hz, CH₃-CF-CH₃), 1.95-2.30(2H, m, OCH₂CH₂), 4.15-4.45(2H, m, OCH₂), 5.20-5.55(1H, m, CH-N), 6.10-6.55(3H, m, NH, NH₂), 6.60-7.35(4H, m, Ar) |
| 40 | 1560 | 1.41(3H, d, J=7.7Hz, OCH-CH₃), 1.61(6H, d, J=22.0Hz, CH₃-CF-CH₃) 2.00-2.50(2H, m, OCHCH₂), 2.23(3H, s, Ar-CH₃), 4.00-4.50(1H, m, OCH), 4.90-5.25(1H, m, CH-N), 5.20-5.75(3H, m, NH, NH₂), 6.60-7.15(3H, m, Ar) |
| 41 | 1540 | 1.33(3H, s, OCCH₃), 1.42(3H, s, OCCH₃), 1.60(6H, d, J=22.0Hz, CH₃-CF-CH₃), 2.19(3H, s, Ar-CH₃), 2.10-2.40(2H, m, OCCH₂), 5.20-5.55(1H, m, CH-N), 6.10-6.50(3H, m, NH, NH₂), 6.50-7.20(3H, m, Ar) |

| | | |
|---|---|---|
| **∗1 Potassium bromide tablet method** | | |
| **∗2 Solvent: Deutero chloroform, Internal standard: Tetramethylsilane (TMS)** | | |

**Table 34**

| **IR◆NMR data** | | |
|---|---|---|
| **Example No.** | **IR(cm**^{**-1**}**)****∗1** **s-triazine** | ^{**1**}**H-NMR*******^{**2**} |
| 42 | 1580 | 1.66(6H, d, J=22.1Hz, CH₃-CF-CH₃) 2.00-2.30(2H, m, OCH₂CH₂), 4.15-4.35(2H, m, OCH₂), 5.05-5.70(4H, m, CH-NH, NH₂), 6.76(1H, d, J=8.6Hz, Ar) 7.13(1H, dd, J=8.6, 2.7Hz, Ar), 7.26(1H, d, J=2.7Hz, Ar) |
| 43 | 1580 | 1.25(9H, s, t-Bu), 1.61(6H, d, J=21.9Hz, CH₃-CF-CH₃), 2.00-2.30(2H, m, OCH₂CH₂), 4.05-4.30(2H, m, OCH₂), 4.95-6.55(4H, m, CH-NH, NH₂), 6.75(1H, d, J=9.5Hz, Ar), 7.10-7.30(2H, m, Ar) |
| 44 | 1570 | 1.64(6H, d, J=21.9Hz, CH₃-CF-CH₃), 1.90-2.30(2H, m, OCH₂CH₂), 2.23(3H, s, Ar-CH₃), 4.10-4.30(2H, m, OCH₂), 4.95-6.15(4H, m, CH-NH, NH₂), 6.71(1H, d, J=8.1Hz, Ar), 6.98(1H, d, J=8.1Hz, Ar), 7.03(1H, s, Ar) |
| 45 | 1580 | 1.64(6H, d, J=22.1Hz, CH₃-CF-CH₃), 2.05-2.30(2H, m, OCH₂CH₂), 2.18(3H, s, Ar-CH₃), 4.15-4.40(2H, m, OCH₂), 5.00-5.80(4H, m, CH-NH, NH₂), 6.77(1H, dd, J=7.6, 7.3Hz, Ar), 7.05(1H, d, J=7.6Hz, Ar), 7.09(1H, d, J=7.3Hz, Ar) |
| 46 | 1580 | 1.64(6H, d, J=21.9Hz, CH₃-CF-CH₃), 2.05-2.35(2H, m, OCH₂CH₂), 2.28(3H, s, Ar-CH₃), 4.10-4.30(2H, m, OCH₂), 5.00-5.90(4H, m, CH-NH, NH₂), 6.65(1H, s, Ar), 6.70(1H, d, J=7.7Hz, Ar), 7.13(1H, d, J=7.7Hz, Ar) |
| 47*³ | 1570 | 1.55(6H, d, J=21.4Hz, CH₃-CF-CH₃), 1.95-2.20(2H, m, OCH₂CH₂), 2.18(3H, s, Ar-CH₃), 4.15-4.30(2H, m, OCH₂), 5.00-5.40(1H, m, CH-NH), 5.90-6.80(3H, m, CH-NH, NH₂), 6.55-6.80(2H, m, Ar), 7.02(1H, d, J=7.0Hz, Ar) |
| 48 | 1570 | 1.65(6H, d, J=22.1Hz, CH₃-CF-CH₃), 2.00-2.30(2H, m, OCH₂CH₂), 2.41(3H, s, SCH₃), 4.10-4.35(2H, m, OCH₂), 5.00-6.10(4H, m, CH-NH, NH₂), 6.77(1H, d, J=8.6Hz, Ar), 7.17(1H, dd, J=8.6, 2.3Hz, Ar), 7.23(1H, d, J=2.3Hz, Ar) |
| 49 | 1580 | 1.18(3H, t, J=7.5Hz, CH₂-CH₃), 1.65(6H, d, J=22.1Hz, CH₃-CF-CH₃), 2.00-2.35(2H, m, OCH₂CH₂), 2.54(2H, q, J=7.5Hz, CH₂-CH₃), 4.05-4.35(2H, m, OCH₂), 5.00-6.00(4H, m, CH-NH, NH₂), 6.74(1H, d, J=7.5Hz, Ar), 7.02(1H, dd, J=7.5, 2.1Hz, Ar), 7.06(1H, d, J=2.1Hz, Ar) |
| 50 | 1570 | 1.65(6H, d, J=21.9Hz, CH₃-CF-CH₃), 2.00-2.30(2H, m, OCH₂CH₂), 4.10-4.30(2H, m, OCH₂), 5.05-6.00(4H, m, CH-NH, NH₂), 6.70(1H, d, J=8.8Hz, Ar), 7.26(1H, dd, J=8.8, 2.4Hz, Ar), 7.39(1H, d, J=2.4Hz, Ar) |

| | | |
|---|---|---|
| **∗1 Potassium bromide tablet method** | | |
| **∗2 Solvent: Deutero chloroform, Internal standard: Tetramethylsilane (TMS)** | | |
| **∗3 Solvent: Deutero acetone, Internal standard: Tetramethylsilane (TMS)** | | |

**Table 35**

| **IR◆NMR data** | | |
|---|---|---|
| **Example No.** | **IR(cm**^{**-1**}**)****∗1** **s-triazine** | ^{**1**}**H-NMR*******^{**2**} |
| 51 | 1580 | 1.64(6H, d, J=21.9Hz, CH₃-CF-CH₃), 2.10-2.35(2H, m, OCH₂CH₂), 4.20-4.40(2H, m, CH₂), 5.10-6.20(4H, m, CH-NH, NH₂), 6.70-7,10(3H, m, Ar), |
| 52 | 1580 | 1.64(6H, d, J=21.9Hz, CH₃-CF-CH₃), 2.10-2.35(2H, m, OCH₂CH₂), 4.25-4.50(2H, m, OCH₂), 5.10-6.00(4H, m, CH-NH, NH₂), 6.81(1H, dd, J=7.7, 7.1Hz, Ar), 7.19(1H, d, J=7.1Hz, Ar), 7.27(1H, d, J=7.7Hz, Ar) |
| 53 | 1570 | 1.64(6H, d, J=21.9Hz, CH₃-CF-CH₃), 2.00-2.35(2H, m, OCH₂CH₂), 4.15-4.35(2H, m, OCH₂), 5.05-6.05(4H, m, CH-NH, NH₂), 6.81(1H, d, J=8.8Hz, Ar), 7.06(1H, d, J=8.8Hz, Ar), 7.14(1H, s, Ar) |
| 54 | 1580 | 1.64(6H, d, J=22.1Hz, CH₃-CF-CH₃), 2.05-2.30(2H, m, OCH₂CH₂), 4.10-4.30(2H, m, OCH₂), 5.00-6.20(4H, m, CH-NH, NH₂), 6.45-6.70(2H, m, Ar), 7.10-7.30(1H, m, Ar) |
| 55 | 1560 | 1.20(3H, t, J=7.6Hz, CH₂-CH₃), 1.67(6H, d, J=21.3Hz, CH₃-CF-CH₃ ), 2.05-2.35(2H, m, OCH₂CH₂), 2.59(2H, q, J=7.6Hz, CH₂-CH₃), 4.00-4.45(2H, m, OCH₂), 5.00-5.40(1H, m, CH-NH), 5.30-5.60(3H, m, NH, NH₂), 6.68(1H, s, Ar), 6.72(1H, d, J=7.3Hz, Ar), 7.17(1H, d, J=7.3Hz, Ar) |
| 56 | 1560 | 1.64(6H, d, J=21.9Hz, CH₃-CF-CH₃), 2.00-2.30(2H, m, OCH₂CH₂), 4.10-4.35(2H, m, OCH₂), 5.00-6.30(4H, m, CH-NH, NH₂), 6.80-7.30(3H, m, Ar), |
| 57 | 1560 | 1.62(6H, d, J=21.7Hz, CH₃-CF-CH₃), 2.17(3H, s, Ar-CH₃), 2.23(3H, s, Ar-CH₃), 2.00-2.40(2H, m, OCH₂CH₂), 4.00-4.35(2H, m, OCH₂), 4.90-5.30(1H, m, CH-NH), 5.80-6.30(3H, m, NH, NH₂), 6.50(1H, s, Ar), 6.69(1H, s, Ar) |
| 58 | 1580 | 1.62(6H, d, J=22.1Hz, CH₃-CF-CH₃) 1.90-2.30(2H, m, OCH₂CH₂), 2.16(3H, s, Ar-CH₃), 2.19(3H, s, Ar-CH₃), 3.85-4.50(2H, m, OCH₂), 4.95-6.10(4H, m, CH-NH, NH₂), 6.65(1H, d, J=7.7Hz, Ar), 6.98(1H, d, J=7.7Hz, Ar) |
| 59 | 1570 | 1.65(6H, d, J=21.9Hz, CH₃-CF-CH₃) 2.05-2.30(2H, m, OCH₂CH₂), 2.16(3H, s, Ar-CH₃), 2.21(3H, s, Ar-CH₃), 4.10-4.35(2H, m, OCH₂), 5.00-5.80(4H, m, CH-NH, NH₂), 6.88(2H, s, Ar) |

| | | |
|---|---|---|
| **∗1 Potassium bromide tablet method** | | |
| **∗2 Solvent: Deutero chloroform, Internal standard, Tetramethylsilane (TMS)** | | |

**Table 36**

| **IR◆NMR data** | | |
|---|---|---|
| **Example No.** | **IR(cm**^{**-1**}**)****∗1** **s-triazine** | ^{**1**}**H-NMR*******^{**2**} |
| 60 | 1580 | 1.63(6H, d, J=22.1Hz, CH₃-CF-CH₃), 1.95-2.30(2H, m, OCH₂CH₂), 2.11(3H, s, Ar-CH₃), 2.23(3H, s, Ar-CH₃), 4.15-4.35(2H, m, OCH₂), 5.00-6.30(4H, m, CH-NH, NH₂), 6.69(1H, d, J=7.9Hz, Ar), 6.99(1H, d, J=7.9Hz, Ar) |
| 61 | 1565 | 1.64(6H, d, J=21.7Hz, CH₃-CF-CH₃), 2.00-2.45(2H, m, OCH₂CH₂), 2.13(3H, s, Ar-CH₃), 2.19(3H, s, Ar-CH₃), 4.00-4.40(2H, m, OCH₂), 5.00-5.70(4H, m, CH-NH, NH₂), 6.62(1H, s, Ar), 7.00(1H, s, Ar) |
| 62 | 1570 | 1.63(6H, d, J=21.9Hz, CH₃-CF-CH₃), 1.90-2.30(2H, m, OCH₂CH₂), 3.86(2H, s, CH₂-Ph), 4.05-4.30(2H, m, OCH₂), 5.00-6.20(4H, m, CH-NH, NH₂), 6.73(1H, d, J=8.2Hz, Ar), 6.90-7.30(7H, m, Ar) |
| 63 | 1580 | 1.65(6H, d, J=22.1Hz, CH₃-CF-CH₃), 2.00-2.35(2H, m, OCH₂CH₂), 3.73(3H, s, OCH₃), 4.10-4.30(2H, m, OCH₂), 5.00-6.00(4H, m, CH-NH, NH₂), 6.76(3H, s, Ar) |
| 64 | 1560 | 1.60(6H, d, J=22.1Hz, CH₃-CF-CH₃), 2.00-2.30(2H, m, OCH₂CH₂), 4.10-4.30(2H, m, OCH₂), 5.00-6.40(4H, m, CH-NH, NH₂), 6.75-7.40(8H, m, Ar) |
| 65 | 1560 | 1.64(6H, d, J=21.9Hz, CH₃-CF-CH₃), 2.10-2.35(2H, m, OCH₂CH₂), 4.15-4.35(2H, m, OCH₂), 5.10-6.00(4H, m, CH-NH, NH₂), 6.90(1H, d, J=8.6Hz, Ar), 7.25-7.60(7H, m, Ar) |
| 66 | 1560 | 1.19(6H, d, J=6.8Hz, CH₃-CH-CH₃), 1.64(6H, d, J=22.1Hz, CH₃-CF-CH₃), 2.00-2.30(2H, m, OCH₂CH₂), 2.81(1H, sept, J=6.8Hz, CH₃-CH-CH₃), 4.10-4.30(2H, m, OCH₂), 5.00-6.20(4H, m, CH-NH, NH₂), 6.76(1H, d, J=9.1Hz, Ar), 7.04(1H, d, J=9.1Hz, Ar), 7.09(1H, s, Ar) |
| 67 | 1580 | 1.18(3H, t, J=7.6Hz, CH₂-CH₃), 1.62(6H, d, J=21.9Hz, CH₃-CF-CH₃), 2.05-2.30(2H, m, OCH₂CH₂), 2.60(2H, q, J=7.6Hz, CH₂-CH₃), 4.15-4.35(2H, m, OCH₂), 5.00-6.40(4H, m, CH-NH, NH₂), 6.80(1H, dd, J=7.5, 7.3Hz, Ar), 6.95-7.20(2H, m, Ar) |
| 68 | 1570 | 1.65(6H, d, J=22.1Hz, CH₃-CF-CH₃), 2.10-2.35(2H, m, OCH₂CH₂), 4.10-4.30(2H, m, OCH₂), 5.10-6.20(4H, m, CH-NH, NH₂), 6.94(1H, dd, J=8.0. 7.0Hz, Ar), 7.10-7.60(7H, m, Ar) |

| | | |
|---|---|---|
| **∗1 Potassium bromide tablet method** | | |
| **∗2 Solvent: Deutero chloroform, Internal standard: Tetramethylsilane (TMS)** | | |

**Table 37**

| **IR◆NMR data** | | |
|---|---|---|
| **Example No.** | **IR(cm**^{**-1**}**)****∗1** **s-triazine** | ^{**1**}**H-NMR*******^{**2**} |
| 69 | 1580 | 1.64(6H, d, J=22.1Hz, CH₃-CF-CH₃, 2.10-2.35(2H, m, OCH₂CH₂), 2.43(3H, s, SCH₃), 4.20-4.50(2H, m, OCH₂), 5.00-6.20(4H, m, CH-NH, NH₂), 6.75-7.15(3H, m, Ar) |
| 70 | 1580 | 1.64(6H, d, J=22.1Hz, CH₃-CF-CH₃), 2.05-2.35(2H, m, OCH₂CH₂), 3.88(3H, s, OCH₃), 4.20-4.45(2H, m, OCH₂), 5.00-6.10(4H, m, CH-NH, NH₂), 6.75-6.95(3H, m, Ar) |
| 71 | 1570 | 1.63(6H, d, J=22.1Hz, CH₃-CF-CH₃), 2.05-2.30(2H, m, OCH₂CH₂), 3.93(2H, s, CH₂-Ph), 4.10-4.35(2H, m, OCH₂), 5.00-6.10(4H, m, CH-NH, NH₂), 6.77(1H, dd, J=7.6, 7.3Hz, Ar), 6.96(1H, dd, J=7.6. 2.3Hz, Ar), 7.11(1H, dd, J=7.3, 2.3Hz, Ar), 7.15-7.30(5H, m, Ar) |
| 72 | 1580 | 1.65(6H, d, J=22.1Hz, CH₃-CF-CH₃), 2.00-2.25(2H, m, OCH₂CH₂), 2.15(3H, s, Ar-CH₃), 4.15-4.35(2H, m, OCH₂), 5.00-6.00(4H, m, CH-NH, NH₂), 7.01(1H, d, J=2.7Hz, Ar), 7.09(1H, d, J=2.7Hz, Ar) |
| 73 | 1560 | 1.64(6H, d, J=22.1Hz, CH₃-CF-CH₃), 2.05-2.45(2H, m, CH₂CH₂), 2.22(3H, s, Ar-CH₃), 4.20-4.40(2H, m, OCH₂), 5.00-6.20(4H, m, CH-NH, NH₂), 7.02(1H, s, Ar), 7.25(1H, s, Ar) |
| 74 | 1560 | 1.68(6H, d, J=21.7Hz, CH₃-CF-CH₃), 2.10-2.35(2H, m, OCH₂CH₂), 4.15-4.40(2H, m, OCH₂), 5.10-5.80(4H, m, CH-NH, NH₂), 7.00-7.65(8H, m, Ar) |
| 75 | 1570 | 1.65(6H, d, J=21.7Hz, CH₃-CF-CH₃), 2.00-2.35(2H, m, OCH₂CH₂), 4.15-4.45(2H, m, OCH₂), 5.10-6.20(4H, m, CH-NH, NH₂), 6.95-7.50(3H, m, Ar) |
| 76 | 1580 | 1.60(3H, dd, J=23.9, 8.1Hz, CH₃-CHF), 2.00-2.35(2H, m, OCH₂CH₂), 4.10-4.40(2H, m, OCH₂), 4.80-5.90(5H, m, CHF, CH-NH, NH₂), 6.70-7.40(4H, m, Ar) |
| 77 | 1580 | 2.05-2.30(2H, m, OCH₂CH₂), 4.15-4.35(2H, m, OCH₂), 5.00-6.00(4H, m, CH-NH, NH₂), 6.77(1H, d, J=8.4Hz, Ar), 7.15(1H, dd, J=8.4, 2.6Hz, Ar), 7.20(1H, d, J=2.6Hz, Ar) |
| 78*⁴ | 1570 | 2.00-2.30(2H, m, OCH₂CH₂), 2.24(3H, s, CH₃-Ar), 4.10-4.30(2H, m, OCH₂), 4.95-5.40(1H, m, CH-NH), 6.72(1H, d, J=9.1Hz, Ar), 7.01(1H, d, J=9.1Hz, Ar), 7.03(1H, s, Ar) |

| | | |
|---|---|---|
| **∗1 Potassium bromide tablet method** | | |
| **∗2 Solvent: Deutero chloroform, Internal standard: Tetramethylsilane (TMS)** | | |
| **∗4 Solvent: Deutero chloroform + deutero methanol, Internal standard: Tetramethylsilane (TMS)** | | |

**Table 38**

| **IR◆NMR data** | | |
|---|---|---|
| **Example No.** | **IR(cm**^{**-1**}**)****∗1** **s-triazine** | ^{**1**}**H-NMR*******^{**2**} |
| 79*⁴ | 1580 | 2.00-2.30(2H, m, OCH₂CH₂), 2.18(3H, s, Ar-CH₃), 4.15-4.40(2H, m, OCH₂), 5.10-5.35(1H, m, CH-NH), 6.79(1H, dd, J=8.2, 7.6Hz, Ar), 7.06(1H, d, J=7.6Hz, Ar), 7.06(1H, d, J=8.2Hz, Ar), |
| 80*⁴ | 1580 | 2.05-2.35(2H, m, OCH₂CH₂), 2.28(3H, s, Ar-CH₃), 4.10-4.30(2H, m, OCH₂), 5.05-5.35(1H, m, CH-NH), 6.66(1H, s, Ar), 6.71(1H, d, J=7.6Hz, Ar), 7.10(1H, d, J=7.6Hz, Ar) |
| 81*³ | 1570 | 1.95-2.25(2H, m, OCH₂CH₂), 2.19(3H, s, Ar-CH₃), 4.15-4.35(2H, m, OCH₂), 5.05-5.35(1H, m, CH-NH), 6.55-6.80(2H, m, Ar), 6.70-7.50(3H, m, NH, NH₂), 7.04(1H, d, J=7.7Hz, Ar) |
| 82 | 1550 | 2.18(3H, s, Ar-CH₃), 2.27(3H, s, Ar-CH₃), 2.00-2.35(2H, m, OCH₂CH₂), 4.05-4.35(2H, m, OCH₂), 5.00-5.30(1H, m, CH-NH), 6.51(1H, s, Ar), 6.58(1H, s, Ar) |
| 83 | 1560 | 1.90-2.60(2H, m, OCH₂CH₂), 2.23(3H, s, Ar-CH₃), 2.25(3H, s, Ar-CH₃), 3.90-4.45(2H, m, OCH₂), 4.95-5.25(1H, m, CH-NH), 6.70(1H, s, Ar), 6.95(1H, s, Ar) |
| 84 | 1560 | 1.20(3H, t, J=7.6Hz, CH₂-CH₃), 2.00-2.40(2H, m, OCH₂CH₂), 2.57(2H, q, J=7.6Hz, CH₂-CH₃), 3.95-4.45(2H, m, OCH₂), 5.00-6.20(4H, m, CH-NH, NH₂), 6.67(1H, s, Ar) 6.72(1H, d, J=7.3Hz, Ar), 7.10(1H, d, J=7.3Hz, Ar) |
| 85 | 1580 | 2.10-2.40(2H, m, OCH₂CH₂), 4.10-4.35(2H, m, OCH₂), 4.90-5.90(5H, m, CHF, CH-NH, NH₂), 6.70-7.35(4H, m, Ar) |
| 86 | 1580 | 0.93(3H, t, J=7.3Hz, CH₃), 1.40-2.30(5H, m, OH, OCH₂CH₂, CH₂-CH₃), 4.10-4.30(3H, m, OCH₂, CH-OH), 4.90-6.00(4H, m, CH-NH, NH₂), 6.72(1H, d, J=8.6Hz, Ar), 7.09(1H, dd, J=8.6, 2.4Hz, Ar), 7.18(1H, d, J=2.4Hz, Ar) |
| 87 | 1570 | 0.85-1.15(3H, m, CH₃), 1.40-2.30(10H, m, OCH₂CH₂, CH₂-CH₃, THP), 3.20-4.40(5H, m, OCH₂, OCH, THP), 4.60-5.90(5H, m, CH-NH, NH₂, THP), 6.72(1H, d, J=8.6Hz, Ar), 7.10(1H, dd, J=8.6, 2.4Hz, Ar), 7.22(1H, d, J=2.4Hz, Ar) |

| | | |
|---|---|---|
| **∗1 Potassium bromide tablet method** | | |
| **∗2 Solvent: Deutero chloroform, Internal standard: Tetramethylsilane (TMS)** | | |
| **∗3 Solvent: Deutero acetone, Internal standard: Tetramethylsilane (TMS)** | | |
| **∗4 Solvent: Deutero chloroform + deutero methanol, Internal standard: Tetramethylsilane (TMS)** | | |

**Table 39**

| **IR◆NMR data** | | |
|---|---|---|
| **Example No.** | **IR(cm**^{**-1**}**)****∗1** **s-triazine** | ^{**1**}**H-NMR*******^{**2**} |
| 88 | 1570 | 2.10-2.40(2H, m, OCH₂CH₂), 4.10-4.40(2H, m, OCH₂), 5.10-5.45(1H, m, CH-NH), 5.50-6.00(3H, m, CH-NH, NH₂), 7.00-7.60(8H, m, Ar) |
| 89 | 1570 | 1.95-2.30(2H, m, OCH₂CH₂), 4.15-4.40(2H, m, OCH₂), 5.10-5.50(1H, m, CH-NH), 6.60-7.40(4H, m, Ar) |
| 90 | 1570 | 2.20-2.45(2H, m, OCH₂CH₂), 4.15-4.50(2H, m, OCH₂), 5.10-6.00(4H, m, CH-NH, NH₂) 7.00-7.50(3H, m, Ar) |
| 91 | 1570 | 1.63(6H, d, J=22.14Hz, CH₃-CF-CH₃), 1.65-1.90(4H, m, -CH₂-CH₂-CH₂-CH₂-) 2.05-2.25(2H, m, -O-CH₂-CH₂-) 2.50-2.80(4H, m, -CH₂-CH₂-CH₂-CH₂-) 4.15-4.35(2H, m, -O-CH₂-CH₂-) 5.00-6.00(4H, m, -NH-CH-, -NH₂) 6.63(1H, d, J=7.83Hz, Ar) 7.00(1H, d, J=7.83Hz, Ar) |
| 92 | 1570 | 1.63(6H, d, J=22.14Hz, CH₃-CF-CH₃), 1.95-2.30(4H, m, -O-CH₂-CH₂-, -CH₂-CH₂-CH₂-) 2.70-3.00(4H, m, -CH₂-CH₂-CH₂-) 4.15-4.35(2H, m, -O-CH₂-CH₂-) 5.00-6.00(4H, m, -NH-CH-, -NH₂) 6.77(1H, d, J=7.83Hz, Ar) 7.05(1H, d, J=7.83Hz, Ar) |
| 93 | 1550 | 1.28(9H, s, t-Bu) 2.15-2.40(2H, m, -O-CH₂-CH₂-) 4.25-4.55(2H, m, -O-CH₂-CH₂-) 5.00-5.60(4H, m, -NH-CH-, -NH₂) 7.25-7.55(4H, m, Ar) 7.65-7.80(1H, m, Ar) 8.10-8.30(1H, m, Ar) |
| 94 | 1590 | 2.10-2.40(2H, m, -O-CH₂-CH₂-) 4.20-4.65(2H, m, -O-CH₂-CH₂-) 5.20-6.00(4H, m, -NH-CH-, NH₂) 7.25-7.35(2H, m, Ar) 7.40-7.60(2H, m, Ar) 7.70-7.85(1H, m, Ar) 8.10-8.30(1H, m, Ar) |

| | | |
|---|---|---|
| **∗1 Potassium bromide tablet method** | | |
| **∗2 Solvent: Deutero chloroform, Internal standard: Tetramethylsilane (TMS)** | | |

**Table 40**

| **IR◆NMR data** | | |
|---|---|---|
| **Example No.** | **IR(cm**^{**-1**}**)****∗1** **s-triazine** | ^{**1**}**H-NMR*******^{**2**} |
| 95 | 1570 | 1.65(6H, d, J=22.05Hz, CH₃-CF-CH₃), 2.15-2.35(2H, m, -O-CH₂-CH₂-) 4.20-4.60(2H, m, -O-CH₂-CH₂-) 5.15-6.00(4H, m, -NH-CH-, -NH₂), 7.32(2H, s-Ar) 7.41-7.59(2H, m, Ar) 7.65-7.85(1H, m, Ar) 8.10-8.25(1H, m, Ar) |
| 96*³ | 1565 | 2.10-2.50(2H, m, -O-CH₂-CH₂) 4.25-4.65(2H, m, -O-CH₂-CH₂) 5.40-6.00(4H, m, -NH-CH-, -NH₂) 6.90-7.20(1H, m, Ar) 7.25-7.65(2H, m, Ar) 7.65-8.00(3H, m, Ar) |
| 97*³ | 1540 | 1.63(6H, d, J=22.08Hz, CH₃-CF-CH₃), 2.05-2.45(2H, m, -O-CH₂-CH₂-) 4.25-4.60(2H, m, -O-CH₂-CH₂-) 5.50-6.60(4H, m, -NH-CH-, -NH₂) 6.80-7.20(1H, m, Ar) 7.25-7.60(2H, m, Ar) 7.60-8.05(3H, m, Ar) |
| 98*⁴ | 1560 | 1.65(6H, d, J=21.9Hz, CH₃-CF-CH₃), 2.10-2.40(2H, m, OCH₂CH₂), 3.05(3H, s, SO₂-CH₂), 4.25-4.45(2H, m, OCH₂), 5.20-5.40(1H, m, -CH-NH), 7.00(1H, d, J=8,6Hz, Ar), 7.72(1H, dd, J=8.6 2.2Hz, Ar), 7.90(1H, d, J=2,2Hz, Ar) |
| 99 | 1560 | 1.65(6H, d, J=21.9Hz, CH₃-CF-CH₃), 2.15-2.40(2H, m, OCH₂CH₂), 9.22(9H, s, SO₂-CH₂), 4.30-4.55(2H, m, OCH₂), 5.15-6.00(4H, m, -CH-NH,-NH₂), 7.04(1H, dd, J=7.7, 7.7Hz, Ar), 7.57(1H, dd, J=7.7, 1.8Hz, Ar), 7.90(1H, dd, J=7.7, 1.8Hz, Ar) |
| 100 | 1570 | 1.65(6H, d, J=22.14.Hz, CH₃-CF-CH₃), 2.10-2.30(2H, m, -O-CH₂-CH₂-) 4.25-4.40(2H, m, -O-CH₂-CH₂-) 5.20-6.10(4H, m, -NH-CH-, -NH₂) 6.87(1H, d, J=8.37Hz, Ar) 7.45(1H, dd, J=8.37, 1.98Hz, Ar) 7.61(1H, d, J=1.98Hz, Ar) |

| | | |
|---|---|---|
| **∗1 Potassium bromide tablet method** | | |
| **∗2 Solvent: Deutero chloroform, Internal standard: Tetramethylsilane (TMS)** | | |
| **∗3 Solvent: Deutero acetone, Internal standard: Tetramethylsilane (TMS)** | | |
| **∗4 Solvent: Deutero chloroform + deutero methanol Internal standard: Tetramethylsilane (TMS)** | | |

### [Herbicide Example]

### (1) Preparation of herbicides

97 Parts by weight of talc (trade name: Zeaklite) as a carrier, 1.5 parts by weight of alkylaryl sulfonate (trade name: Neoplex, supplied by Kao-Atlas K.K.) as a surfactant and 1.5 parts by weight of a nonionic and anionic surfactant (trade name: Sorpol 800A, supplied by Toho Chemical Co., Ltd.) were uniformly pulverized and mixed to prepare a carrier for a wettable powder.

90 Parts by weight of the above carrier for a wettable powder and 10 parts by weight of one of the compounds of the present invention, obtained in Examples 1 to 3, 5 to 13, 15, 16, 32 to 90, 98 and 99 (Example Numbers are used as numbers of the compounds), were uniformly pulverized and mixed to obtain herbicides.

### (2) Post-emergence treatment test

Seeds of weeds such as cocklebur, velvetleaf, ivyleaf morningglory, pale smartweed, jimsonweed, rough pigweed and black nightshade and seeds of cotton were sown in 1/5,000-are Wagner pots filled with upland soil, and covered with upland soil. The seeds were grown in a greenhouse, and at the stage of 1 ∼ 2 leaves of these plants, a predetermined amount of the herbicide prepared in the above (1) was suspended in water, and the suspension was uniformly sprayed onto leaf and stalk portions at a rate of 2,000 liters/hectare. Then, the plants were grown in the greenhouse, and on the 20th day after the treatment, the herbicide was evaluated for herbicidal efficacy and phytotoxicity to the crop. Tables 41 to 44 show the results.

The herbicidal efficacy and the phytotoxicity are shown according to the following ratings.

### (Ratings)

| Herbicidal efficacy | Ratio of remaining plant weight to plant weight in non-treated plot (%) |
|---|---|
| 0 | 81 - 100 |
| 1 | 61 - 80 |
| 2 | 41 - 60 |
| 3 | 21 - 40 |
| 4 | 1 - 20 |
| 5 | 0 |

| Phytotoxicity | Ratio of remaining plant weight to plant weight in non-treated plot (%) |
|---|---|
| - | 100 |
| ± | 95 - 99 |
| + | 90 - 94 |
| ++ | 80 - 89 |
| +++ | 0 - 79 |

The ratio of remaining plant weight to plant weight in non-treated plot was determined on the basis of the ratio of remaining plant weight to plant weight in non-treated plot = (remaining plant weight in treated plot/plant weight in non-treated plot) x 100.

**Table 41**

| Post-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 2 | 1,000 | 5 | 5 | 4 | 5 | 5 | - | 5 | - |
| Compound 5 | " | 5 | 5 | 5 | 5 | 5 | 5 | 5 | ± |
| Compound 6 | " | 2 | 3 | 5 | 5 | 5 | 5 | 5 | ± |
| Compound 7 | " | 5 | 5 | 5 | 5 | 5 | 5 | 5 | - |
| Compound 8 | " | 5 | 5 | 5 | 5 | 5 | 5 | 5 | + |
| Compound 9 | " | 2 | 5 | 5 | 5 | 5 | 5 | 5 | - |
| Compound 10 | " | 5 | 5 | 5 | 5 | 5 | 5 | 5 | ± |
| Compound 11 | " | 5 | 2 | 5 | 5 | 5 | 5 | 5 | - |
| Compound 12 | " | 5 | 5 | 5 | 5 | 5 | 5 | 5 | + |
| Compound 13 | " | 5 | 5 | 2 | 5 | 5 | 5 | 5 | - |
| Compound 15 | " | 5 | 5 | 3 | 5 | 5 | 4 | 5 | - |
| Compound 16 | " | 4 | 5 | 5 | 5 | 5 | 5 | 5 | + |
| AA = Cocklebur, BB = velvetleaf, CC = Ivyleaf morningglory, DD = Pale smartweed, EE: Jimsonweed, FF: Rough pigweed, GG = Black nightshade | | | | | | | | | |

**Table 42**

| Post-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 32 | 1,000 | 5 | 5 | 5 | 5 | 5 | 5 | 5 | ± |
| Compound 33 | " | 5 | 5 | 3 | 5 | 5 | 5 | 5 | ± |
| Compound 34 | " | 5 | 5 | 2 | 5 | 5 | 5 | 5 | - |
| Compound 35 | " | 5 | 5 | 2 | 5 | 5 | 5 | 5 | - |
| Compound 36 | " | 3 | 3 | 2 | 3 | 3 | 3 | 3 | - |
| Compound 37 | " | 4 | 5 | 3 | 4 | 4 | 5 | 5 | - |
| Compound 38 | " | 3 | 4 | 4 | 5 | 5 | 5 | 5 | ± |
| Compound 39 | " | 4 | 3 | 5 | 5 | 5 | 4 | 4 | - |
| Compound 40 | " | 4 | 2 | 2 | 5 | 5 | 2 | 3 | - |
| Compound 42 | " | 4 | 5 | 4 | 5 | 5 | 5 | 5 | ± |
| Compound 44 | " | 2 | 3 | 3 | 5 | 5 | 5 | 5 | - |
| Compound 45 | " | 3 | 5 | 5 | 5 | 5 | 5 | 5 | ± |
| Compound 46 | " | 5 | 5 | 5 | 5 | 5 | 5 | 5 | + |
| Compound 48 | " | 3 | 3 | 2 | 5 | 2 | 2 | 2 | - |
| Compound 49 | " | 3 | 4 | 2 | 5 | 2 | 2 | 5 | - |
| Compound 50 | " | 5 | 5 | 5 | 5 | 3 | 5 | 5 | - |
| Compound 51 | " | 3 | 3 | 2 | 4 | 2 | 5 | 5 | - |
| Compound 52 | " | 4 | 4 | 2 | 5 | 4 | 5 | 5 | ± |
| Compound 53 | " | 5 | 4 | 2 | 5 | 3 | 4 | 5 | - |
| Compound 54 | " | 5 | 5 | 4 | 5 | 5 | 5 | 5 | - |
| AA = Cocklebur, BB = velvetleaf, CC = Ivyleaf morningglory, DD = Pale smartweed, EE: Jimsonweed, FF: Rough pigweed, GG = Black nightshade | | | | | | | | | |

**Table 43**

| Post-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 55 | 1,000 | 3 | 3 | 3 | 5 | 4 | 4 | 5 | ± |
| Compound 56 | " | 5 | 5 | 2 | 5 | 5 | 4 | 5 | - |
| Compound 59 | " | 5 | 4 | 3 | 5 | 5 | 2 | 5 | ± |
| Compound 60 | " | 4 | 3 | 3 | 5 | 5 | 4 | 5 | - |
| Compound 61 | " | 4 | 5 | 3 | 5 | 4 | 3 | 3 | - |
| Compound 62 | " | 2 | 5 | 3 | 4 | 2 | 3 | 4 | - |
| Compound 63 | " | 5 | 3 | 3 | 5 | 4 | 4 | 5 | ± |
| Compound 64 | " | 3 | 4 | 4 | 4 | 3 | 3 | 4 | - |
| Compound 65 | " | 4 | 4 | 4 | 5 | 4 | 3 | 5 | ± |
| Compound 66 | " | 2 | 3 | 2 | 3 | 3 | 5 | 4 | - |
| Compound 67 | " | 4 | 4 | 3 | 4 | 4 | 5 | 4 | - |
| Compound 69 | " | 3 | 3 | 3 | 4 | 3 | 2 | 4 | - |
| Compound 70 | " | 3 | 2 | 2 | 4 | 3 | 5 | 5 | - |
| Compound 72 | " | 5 | 4 | 4 | 5 | 5 | 3 | 5 | - |
| Compound 73 | " | 3 | 5 | 2 | 5 | 5 | 5 | 5 | - |
| Compound 74 | " | 3 | 3 | 3 | 3 | 2 | 3 | 3 | - |
| Compound 75 | " | 3 | 3 | 2 | 5 | 4 | 4 | 3 | - |
| Compound 76 | " | 5 | 5 | 4 | 5 | 5 | 4 | 5 | - |
| Compound 77 | " | 3 | 2 | 2 | 5 | 5 | 5 | 5 | - |
| Compound 78 | " | 3 | 3 | 4 | 5 | 4 | 4 | 5 | - |
| Compound 79 | " | 2 | 2 | 3 | 5 | 5 | 5 | 5 | ± |
| AA = Cocklebur, BB = velvetleaf, CC = Ivyleaf morningglory, DD = Pale smartweed, EE: Jimsonweed, FF: Rough pigweed, GG = Black nightshade | | | | | | | | | |

**Table 44**

| Post-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 80 | 1,000 | 3 | 4 | 3 | 5 | 5 | 5 | 5 | - |
| Compound 82 | " | 5 | 2 | 2 | 4 | 2 | 2 | 5 | - |
| Compound 83 | " | 3 | 4 | 3 | 5 | 5 | 3 | 5 | - |
| Compound 85 | " | 5 | 5 | 5 | 5 | 5 | 5 | 5 | - |
| Compound 86 | " | 3 | 2 | 3 | 5 | 5 | 5 | 4 | - |
| Compound 88 | " | 3 | 3 | 3 | 3 | 2 | 3 | 3 | - |
| Compound 89 | " | 2 | 2 | 2 | 5 | 4 | 5 | 5 | - |
| Compound 90 | " | 2 | 2 | 2 | 5 | 4 | 5 | 4 | - |
| Compound 99 | " | 3 | 3 | 3 | 4 | 5 | 4 | 5 | - |
| AA = Cocklebur, BB = velvetleaf, CC = Ivyleaf morningglory, DD = Pale smartweed, EE: Jimsonweed, FF: Rough pigweed, GG = Black nightshade | | | | | | | | | |

The results in Tables 41 to 44 show that the herbicide containing the triazine derivative of the present invention can control a broad range of upland weeds at a low dosage without causing phytotoxicity on cotton in post-emergence treatment. Above all, Compounds 2, 7, 9, 13, 15, 34, 35, 37, 50, 54, 61, 62, 73, 76 and 85 exhibit high safety for cotton and exhibit high herbicidal efficacy against velvetleaf belonging to malvaceous weeds to which cotton also belongs, and they particularly have excellent inter-genus selectivity.

### (3) Upland soil pre-emergence treatment test

Seeds of weeds such as cocklebur, velvetleaf, ivyleaf morningglory, jimsonweed, rough pigweed, green foxtail and large crabgrass and seeds of cotton were sown in 1/5,000-are Wagner pots filled with upland soil, and covered with upland soil. Then, a predetermined amount of the herbicide prepared in the above (1) was suspended in water and uniformly sprayed onto the soil surface. Then, the seeds were grown in a greenhouse, and on the 20th day after the treatment, the herbicide was evaluated for herbicidal efficacy and phytotoxicity to the crop. Tables 45 to 48 show the results.

The data of the herbicidal efficacy and phytotoxicity to the crop are shown on the basis of the ratings shown in the (2) post-emergence treatment test.

**Table 45**

| Upland soil pre-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 1 | 3,000 | 5 | 5 | 5 | 5 | 5 | 5 | 5 | - |
| Compound 2 | " | 5 | 5 | 5 | 5 | 5 | 5 | 5 | - |
| Compound 3 | " | 5 | 5 | 5 | 5 | 5 | 5 | 5 | + |
| Compound 5 | " | 3 | 5 | 2 | 5 | 5 | 5 | 5 | + |
| Compound 8 | " | 3 | 5 | 5 | 5 | 5 | 5 | 5 | ± |
| Compound 10 | " | 5 | 5 | 5 | 5 | 5 | 5 | 5 | + |
| Compound 12 | " | 5 | 5 | 5 | 5 | 5 | 5 | 5 | + |
| Compound 13 | " | 3 | 5 | 5 | 5 | 5 | 5 | 5 | - |
| Compound 15 | " | 5 | 5 | 5 | 5 | 5 | 5 | 5 | - |
| Compound 16 | " | 5 | 5 | 5 | 5 | 5 | 5 | 5 | + |
| AA = Cocklebur, BB = velvetleaf, CC = Ivyleaf morningglory, DD = Jimsonweed, EE = rough pigweed, FF = Green foxtail, GG = Large crabgrass | | | | | | | | | |

**Table 46**

| Upland soil pre-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 32 | 3,000 | 5 | 5 | 5 | 5 | 5 | 5 | 5 | + |
| Compound 33 | " | 5 | 5 | 5 | 5 | 5 | 5 | 5 | ± |
| Compound 34 | " | 3 | 5 | 4 | 5 | 5 | 5 | 2 | - |
| Compound 35 | " | 5 | 5 | 5 | 5 | 5 | 5 | 5 | - |
| Compound 36 | " | 3 | 4 | 3 | 3 | 3 | 3 | 4 | - |
| Compound 37 | " | 3 | 5 | 3 | 3 | 5 | 3 | 4 | - |
| Compound 38 | " | 5 | 4 | 5 | 5 | 5 | 4 | 5 | - |
| Compound 39 | " | 5 | 5 | 3 | 5 | 5 | 5 | 4 | ± |
| Compound 40 | " | 5 | 2 | 2 | 5 | 5 | 5 | 5 | - |
| Compound 41 | " | 3 | 2 | 2 | 3 | 5 | 3 | 3 | - |
| Compound 42 | " | 5 | 5 | 5 | 5 | 5 | 5 | 5 | + |
| Compound 43 | " | 3 | 3 | 3 | 3 | 4 | 3 | 3 | - |
| Compound 44 | " | 5 | 5 | 4 | 5 | 5 | 5 | 5 | - |
| Compound 45 | " | 3 | 5 | 3 | 5 | 5 | 4 | 5 | ± |
| Compound 46 | " | 5 | 5 | 4 | 5 | 5 | 5 | 5 | - |
| Compound 47 | " | 4 | 4 | 3 | 4 | 5 | 3 | 3 | + |
| Compound 48 | " | 5 | 5 | 3 | 5 | 5 | 5 | 5 | - |
| Compound 49 | " | 5 | 5 | 3 | 5 | 5 | 5 | 5 | - |
| Compound 50 | " | 5 | 5 | 3 | 5 | 5 | 5 | 5 | - |
| Compound 51 | " | 5 | 5 | 4 | 5 | 5 | 5 | 5 | + |
| AA = Cocklebur, BB = velvetleaf, CC = Ivyleaf morningglory, DD = Jimsonweed, EE = rough pigweed, FF = Green foxtail, GG = Large crabgrass | | | | | | | | | |

**Table 47**

| Upland soil pre-emergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 52 | 3,000 | 5 | 5 | 3 | 5 | 5 | 5 | 5 | - |
| Compound 53 | " | 3 | 5 | 3 | 5 | 5 | 2 | 5 | - |
| Compound 54 | " | 5 | 5 | 4 | 4 | 5 | 3 | 3 | - |
| Compound 55 | " | 4 | 5 | 4 | 5 | 5 | 5 | 5 | ± |
| Compound 56 | " | 3 | 4 | 3 | 4 | 5 | 5 | 5 | - |
| Compound 57 | " | 3 | 3 | 3 | 5 | 5 | 3 | 3 | - |
| Compound 58 | " | 3 | 3 | 3 | 4 | 5 | 3 | 3 | - |
| Compound 59 | " | 3 | 5 | 3 | 5 | 5 | 5 | 5 | - |
| Compound 60 | " | 3 | 5 | 3 | 4 | 5 | 5 | 5 | - |
| Compound 61 | " | 3 | 5 | 3 | 5 | 5 | 5 | 5 | ± |
| Compound 62 | " | 3 | 3 | 3 | 4 | 4 | 4 | 4 | - |
| Compound 63 | " | 3 | 3 | 4 | 4 | 5 | 5 | 5 | ± |
| Compound 64 | " | 3 | 5 | 4 | 5 | 5 | 5 | 5 | ± |
| Compound 65 | " | 4 | 5 | 4 | 4 | 5 | 5 | 4 | - |
| Compound 66 | " | 4 | 3 | 4 | 4 | 3 | 3 | 3 | - |
| Compound 67 | " | 3 | 4 | 3 | 4 | 5 | 4 | 4 | - |
| Compound 68 | " | 3 | 4 | 3 | 4 | 5 | 4 | 4 | - |
| Compound 69 | " | 4 | 3 | 3 | 4 | 4 | 4 | 4 | - |
| Compound 70 | " | 3 | 5 | 3 | 5 | 5 | 5 | 4 | ± |
| Compound 71 | " | 3 | 5 | 3 | 5 | 5 | 5 | 5 | - |
| AA = Cocklebur, BB = velvetleaf, CC = Ivyleaf morningglory, DD = Jimsonweed, EE = rough pigweed, FF = Green foxtail, GG = Large crabgrass | | | | | | | | | |

**Table 48**

| Upland soil preemergence treatment test | | | | | | | | | |
|---|---|---|---|---|---|---|---|---|---|
| Active ingredient in herbicide No. | Dosage g/ha | Herbicidal Efficacy | | | | | | | Phytotoxicity to cotton |
| | | AA | BB | CC | DD | EE | FF | GG | |
| Compound 72 | 3,000 | 3 | 3 | 3 | 4 | 4 | 4 | 3 | - |
| Compound 73 | " | 4 | 5 | 4 | 5 | 5 | 5 | 5 | - |
| Compound 74 | " | 3 | 3 | 3 | 3 | 3 | 3 | 3 | - |
| Compound 75 | " | 3 | 3 | 3 | 4 | 5 | 4 | 3 | - |
| Compound 76 | " | 5 | 5 | 5 | 5 | 5 | 5 | 5 | + |
| Compound 77 | " | 4 | 4 | 3 | 4 | 5 | 3 | 3 | - |
| Compound 78 | " | 3 | 5 | 5 | 5 | 5 | 5 | 5 | ± |
| Compound 79 | " | 3 | 4 | 5 | 5 | 5 | 4 | 4 | - |
| Compound 80 | " | 3 | 5 | 4 | 5 | 5 | 5 | 5 | - |
| Compound 81 | " | 2 | 3 | 3 | 4 | 3 | 3 | 3 | - |
| Compound 82 | " | 5 | 4 | 4 | 4 | 5 | 3 | 3 | - |
| Compound 83 | " | 3 | 3 | 3 | 3 | 5 | 3 | 4 | - |
| Compound 84 | " | 4 | 5 | 3 | 5 | 5 | 5 | 5 | - |
| Compound 85 | " | 4 | 3 | 3 | 3 | 5 | 3 | 3 | - |
| Compound 86 | " | 3 | 5 | 3 | 5 | 5 | 5 | 5 | - |
| Compound 87 | " | 3 | 3 | 3 | 3 | 5 | 3 | 3 | - |
| Compound 88 | " | 3 | 3 | 3 | 3 | 3 | 3 | 3 | - |
| Compound 89 | " | 5 | 5 | 4 | 5 | 5 | 5 | 5 | - |
| Compound 90 | " | 3 | 3 | 3 | 4 | 5 | 3 | 3 | - |
| Compound 98 | " | 5 | 3 | 5 | 3 | 5 | 3 | 3 | ± |
| Compound 99 | " | 3 | 3 | 3 | 3 | 4 | 3 | 3 | - |
| AA = Cocklebur, BB = velvetleaf, CC = Ivyleaf morningglory, DD = Jimsonweed, EE = rough pigweed, FF = Green foxtail, GG = Large crabgrass | | | | | | | | | |

The results in Tables 45 to 48 show that the herbicide containing the triazine derivative of the present invention can control a broad range of upland weeds at a low dosage without causing phytotoxicity on cotton in post-emergence treatment. Above all, Compounds 1, 2, 13, 15, 34, 35, 37, 44, 46, 48, 49, 50, 52, 53, 54, 59, 60, 65, 71, 73, 80, 84, 86 and 89 exhibit high safety for cotton and exhibit high herbicidal efficacy against velvetleaf belonging to malvaceous weeds to which cotton also belongs, and they particularly have excellent inter-genus selectivity.

### Industrial Utility

The triazine derivative of the present invention causes no phytotoxicity on cotton and can selectively control, at a low dosage, a broad range of weeds including velvetleaf belonging to malvaceous weeds to which cotton also belongs, and the triazine derivative of the present invention is therefore remarkably effective as an active ingredient for a herbicide for application to cotton fields.

## Claims

1. A triazine derivative of the general formula (I), 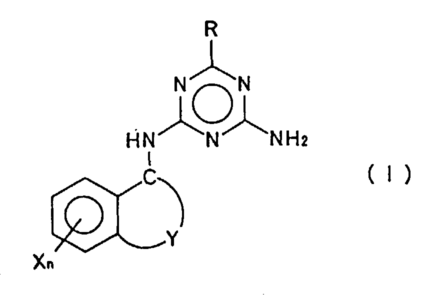
wherein X is a halogen atom, a hydroxyl group, a cyano group, a C₁-C₆ alkyl group, a C₁-C₄ alkoxy group, a C₁-C₄ alkylthio group, a C₁-C₄ alkylsulfonyl group, a C₁-C₆ haloalkyl group, a C₁-C₄ haloalkoxy group, a phenyl-substituted C₁-C₄ alkyl group, a phenyl group or a phenoxy group, provided that when the number of X is plural, plural substituents X may be the same as, or different from, each other or two vicinal substituents X may form a saturated or unsaturated five-membered or six-membered ring together with a carbon-carbon bond in a benzene ring, n is an integer of 0 or 1 to 4,
R is
(1) a C₁-C₆ alkyl group or
(2) a substituted C₁-C₆ alkyl group having 1 to 13 substituents of one or two kinds selected from the class consisting of
i) a halogen atom
ii) a hydroxyl group and
iii) a C₁-C₈ alkoxy group whose alkyl portion may contain a hetero atom, and
Y is a C₂-C₄ alkylene group which may be substituted with 1 to 8 C₁-C₆ alkyl groups or a divalent group of the formula (a), 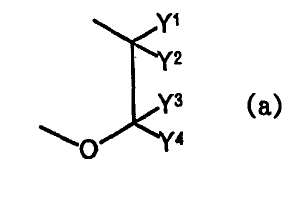
in which each of Y¹ to Y⁴ is independently a hydrogen atom or a C₁-C₄ alkyl group.

2. The triazine derivative of claim 1, wherein X is a C₁-C₄ alkyl group or a halogen atom.

3. The triazine derivative of claim 1, wherein X is selected from the class consisting of methoxy, methylthio, methylsulfonyl, trifluoromethyl, trifluoromethoxy, phenoxyethyl, phenyl and phenoxy.

4. The triazine derivative of claim 2, wherein n is an integer of 1 or 2.

5. The triazine derivative of claim 4, wherein, when n is 2, each of two substituents X are independently a C₁-C₆ alkyl group or a halogen atom.

6. The triazine derivative of claim 1, wherein n is 0.

7. The triazine derivative of claim 1, wherein Y is a propylene group on which one C₁-C₄ alkyl group is subsituted.

8. The triazine derivative of claim 7, wherein Y is a propylene group on which methyl is substituted.

9. The triazine derivative of claim 1, wherein Y is a divalent group of the formula (a), 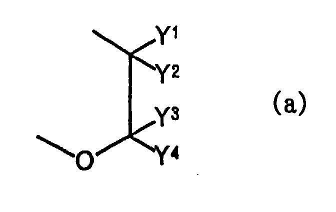
in which each of Y¹ to Y⁴ is independently a hydrogen atom or a C₁-C₄ alkyl group.

10. The triazine derivative of claim 9, wherein each of Y¹ to Y⁴ is independently a hydrogen atom or methyl.

11. The triazine derivative of claim 1, wherein R is a C₁-C₆ alkyl group.

12. The triazine derivative of claim 1, wherein R is a C₁-C₆ alkyl group on which a fluorine atom, a chlorine atom or a bromine atom is substituted.

13. The triazine derivative of claim 12, wherein R is selected from the class consisting of -CF₃, -CCl₃, -CH₂F, -CH₂Cl, -CH₂Br, -C₂F₅, -CH₂CH₂F, -CHF(CH₃), -CHCl(CH₃), -CHBr(CH₃), -CHF(CF₃), -CF(CH₃)₂, -CCl(CH₃)₂, -CBr(CH₃)₂, -CHF(CH₂CH₃), -CHCl(CH₂CH₃) and -CHBr(CH₂CH₃) groups.

14. The triazine derivative of claim 1, wherein R is a C₁-C₆ alkyl group on which a hydroxyl group is substituted.

15. The triazine derivative of claim 14, wherein R is selected from the class consisting of -CH₂OH, -C₂H₄OH, -CH(OH)CH₃, -CH(OH)C₂H₅, -C(CH₃)₂OH and -C(CH₃)₂CH₂OH groups.

16. The triazine derivative of claim 1, wherein R is a C₁-C₆ alkyl group substituted with a group in which a heterocyclic group containing an oxygen atom and an oxygen atom bond to each other.

17. A process for the production of a triazine derivative of the general formula (I), 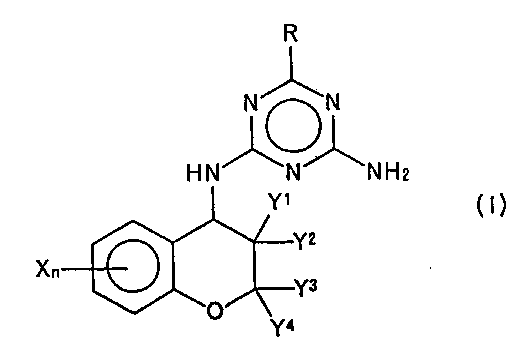
wherein X is a halogen atom, a hydroxyl group, a cyano group, a C₁-C₆ alkyl group, a C₁-C₄ alkoxy group, a C₁-C₄ alkylthio group, a C₁-C₄ alkylsulfonyl group, a C₁-C₆ haloalkyl group, a C₁-C₄ haloalkoxy group, a phenyl-substituted C₁-C₄ alkyl group, a phenyl group or a phenoxy group, provided that when the number of X is plural, plural substituents X may be the same as, or different from, each other or two vicinal substituents X may form a saturated or unsaturated five-membered or six-membered ring together with a carbon-carbon bond in a benzene ring, n is an integer of 0 or 1 to 4,
R is
(1) a C₁-C₆ alkyl group or
(2) a substituted C₁-C₆ alkyl group having 1 to 13 substituents of one or two kinds selected from the class consisting of
i) a halogen atom
ii) a hydroxyl group and
iii) a C₁-C₈ alkoxy group whose alkyl portion may contain a hetero atom, and
Y is a C₂-C₄ alkylene group which may be substituted with 1 to 8 C₁-C₆ alkyl groups or a divalent group of the formula (a), 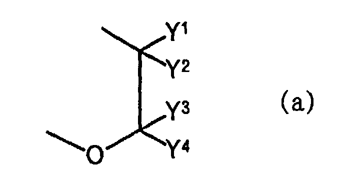
in which each of Y¹ to Y⁴ is independently a hydrogen atom or a C₁-C₄ alkyl group,
which comprises reacting a compound of the general formula (II), 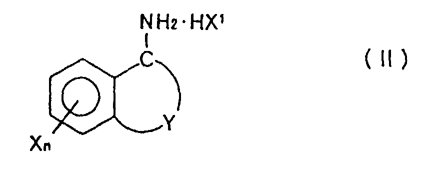
wherein X, n and Y are as defined above and X¹ is a halogen atom,
with cyanoguanidine of the formula (III), 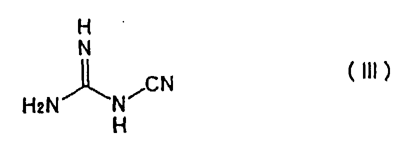 and then reacting the reaction product with an ester of the general formula (IV),
RCOOR¹ (IV)
wherein R is as defined above and R¹ is a C₁-C₄ alkyl group.

18. A herbicide containing the triazine derivative of the general formula (I) recited in claim 1 or a salt thereof as an active ingredient.
